# EUROPEAN PATENT SPECIFICATION

(11) **EP 4 284 843 B1**
(45) Date of publication and mention of the grant of the patent: **20.08.2025**
(21) Application number: 22703329.7
(22) Date of filing: 28.01.2022
(51) Int. Cl.: C08B 37/00, A61K 31/702, A61K 31/715

(54) **NOVEL SIALOSIDES AND THEIR USE IN THERAPY**
NEUARTIGE SIALOSIDE UND DEREN VERWENDUNG IN DER THERAPIE
NOUVEAUX SIALOSIDES ET LEUR UTILISATION EN THÉRAPIE

(30) Priority: 29.01.2021 EP 21305131
(43) Date of publication of application: 06.12.2023
(73) Proprietor: Centre National de la Recherche Scientifique (CNRS), 75016 Paris (FR); Ecole Normale Supérieure de Lyon, 69007 Lyon (FR); Institut National de la Santé et de la Recherche Médicale (INSERM), 75013 Paris (FR); Université Claude Bernard Lyon 1, 69100 Villeurbanne (FR)
(72) Inventor: SAMAIN, Eric, 38610 GIERES (FR); ROSA-CALATRAVA, Manuel, 69003 LYON (FR); TRAVERSIER, Aurélien, 69200 VENISSIEUX (FR); RICHARD, Emeline, 38180 SEYSSINS (FR)
(74) Representative: Novagraaf Technologies
(86) International application number: PCT/EP2022/051981
(87) International publication number: WO 2022/162111

(56) References cited:
- WO-A1-2018/098342
- WO-A2-2005/037187
- "TOPICS IN CURRENT CHEMISTRY.", vol. 367, 1 January 2014 (2014-01-01), DE, pages 125 - 149, XP055814442, ISSN: 0340-1022, Retrieved from the Internet <URL:http://dx.doi.org/10.1007/128_2014_602> DOI: 10.1007/128_2014_602
- HE YUN ET AL: "Neuraminidase Resistant Sialosides for the Detection of Influenza Viruses", vol. 27, no. 6, 15 June 2016 (2016-06-15), US, pages 1509 - 1517, XP055814766, ISSN: 1043-1802, Retrieved from the Internet <URL:https://pubs.acs.org/doi/pdf/10.1021/acs.bioconjchem.6b00150> DOI: 10.1021/acs.bioconjchem.6b00150
- MOCHALOVA L ET AL: "Oligosaccharide specificity of influenza H1N1 virus neuraminidases", ARCHIVES OF VIROLOGY ; OFFICIAL JOURNAL OF THE VIROLOGY DIVISIONOF THE INTERNATIONAL UNION OF MICROBIOLOGICAL SOCIETIES, SPRINGER-VERLAG, VI, vol. 152, no. 11, 6 August 2007 (2007-08-06), pages 2047 - 2057, XP019565403, ISSN: 1432-8798, DOI: 10.1007/S00705-007-1024-Z
- LI XUEBING ET AL: "Carbohydrate-Functionalized Chitosan Fiber for Influenza Virus Capture", vol. 12, no. 11, 1 November 2011 (2011-11-01), pages 3962 - 3969, XP008152713, ISSN: 1525-7797, Retrieved from the Internet <URL:http://pubs.acs.org/journals/bomaf6/index.html> [retrieved on 20111006], DOI: 10.1021/BM200970X
- COTTAZ ET AL: "Genetic engineering of Escherichia coli for the production of N^I,N^I^I-diacetylchitobiose (chitinbiose) and its utilization as a primer for the synthesis of complex carbohydrates", METABOLIC ENGINEERING, ACADEMIC PRESS, US, vol. 7, no. 4, 1 July 2005 (2005-07-01), pages 311 - 317, XP005052642, ISSN: 1096-7176, DOI: 10.1016/J.YMBEN.2005.05.004
- REUTER J D ET AL: "INHIBITION OF VIRAL ADHESION AND INFECTION BY SIALIC-ACID- CONJUGATED DENDRITIC POLYMERS", BIOCONJUGATE CHEMISTRY, AMERICAN CHEMICAL SOCIETY, US, vol. 10, no. 2, 1 March 1999 (1999-03-01), pages 271 - 278, XP000804253, ISSN: 1043-1802, DOI: 10.1021/BC980099N
- ILONA PAPP ET AL: "Inhibition of Influenza Virus Infection by Multivalent Sialic-Acid-Functionalized Gold Nanoparticles", SMALL, vol. 6, no. 24, 20 December 2010 (2010-12-20), pages 2900 - 2906, XP055137473, ISSN: 1613-6810, DOI: 10.1002/smll.201001349
- FIERFORT N ET AL: "Genetic engineering of Escherichia coli for the economical production of sialylated oligosaccharides", JOURNAL OF BIOTECHNOLOGY, ELSEVIER, AMSTERDAM NL, vol. 134, no. 3-4, 30 April 2008 (2008-04-30), pages 261 - 265, XP022588000, ISSN: 0168-1656, [retrieved on 20080310], DOI: 10.1016/J.JBIOTEC.2008.02.010

## Description

### FIELD OF THE INVENTION

The present invention relates to synthetic sialic acid-containing carbohydrates, also designated as sialosides. These novel compounds can be produced by fermentation. These sialosides are able to bind to surface proteins of some viruses, in particular to hemagglutinins of the influenza viruses. Consequently, these sialosides inhibit adhesion of viruses to host cells, and so are useful compounds for preventing or treating infections by viruses.

### BACKGROUND OF THE INVENTION

Viruses have evolved to enter cells from all three domains of life: Bacteria, Archaea and Eukaryotes. Of more than 3,600 known viruses, hundreds can infect human cells and most of those are associated with disease.

Virus entry into animal cells is initiated by attachment to receptors, and is followed by important conformational changes of viral proteins, penetration through or fusion with cellular membranes.

Some viruses, such as influenza viruses, can cross the endosomal membranes very rapidly, and the efficiency of entry can be more than 50% (i.e. 50% of attached viruses do enter cells).

A way to prevent or at least to reduce the viral infection of cells by pathogenic viruses is to inhibit and/or block the interaction of virus with host cells receptors. Studies on these interactions virus / host cells is therefore of primary importance.

Among the virus of the *Orthomyxoviridae* family, the influenza virus is one of the most studied. The infection cycle of influenza virus is initiated by the interaction of the viral hemagglutinin protein (HA) with sialic acid containing glycans (sialosides) incorporated to glycoproteins and glycolipids that are present at the surface of the host cells.

It has been proposed to inhibit the influenza virus adhesion to host cells by saturating the binding sites of the viral hemagglutinin HA with synthetic ligands comprising sialic acid (Matrosovich and Klenk, 2003).

This promising approach is currently pursued, in particular by investigation on the most appropriate synthetic ligands, i.e. those presenting the better affinity for hemagglutinin of each influenza virus.

Searches on this interaction of hemagglutinin of influenza virus and sialosides have shown that influenza viruses have specificities to their host cells via a selective binding to specific sialosides.

Indeed, human and animal influenza viruses present hemagglutinins with different binding preferences for various sialosides expressed on the surface of host cells.

Avian influenza virus strains express hemagglutinins with binding preference to sialic acids linked to the rest of the glycan chain by an α2-3 linkage. In contrast, hemagglutinins from human influenza virus strains show enhanced binding to α2-6-linked sialic acids. This correlates with an abundance of α2-6-linked sialic acids in the upper respiratory tract of humans, and with presence of α2-3-linked sialic acids in the intestinal mucosa of birds, where replication of human and avian strains of influenza viruses takes place, respectively.

Globally, sialic acid linkage specificities influence host range: avian and equine strains of influenza virus recognize sialic acid in an α2,3 linkage to galactose, while human strains prefer sialic acid in an α2,6 linkage. The determinants of influenza tropism are however more complex than only the preferential binding to α2-6 and α2-3 sialic acids.

Human respiratory epithelial cells were shown to express sialylated N-glycans with extended poly-N-acetyllactosamine (poly-LacNAc) chains and it has been suggested that human HAs bind preferentially to extended α2-6 sialosides.

This was confirmed by glycan microarray studies with synthetic sialosides, showing that the human influenza virus HAs from subtypes H1, H2 and H3 bound best to the linear sialosides with di- and tri-LacNAc extensions (Nycholat *et al.,* 2012).

Interestingly, receptors specificity of HA from H3 influenza virus subtype seems to have evolved in recent years: the "earliest" H3 viruses having a preference for short, branched sialosides, while "recent" H3 viruses bind only to linear sialosides with tri-LacNAc extensions (Yang *et al.,* 2015).

It is now well established that not all sialosides bind with equal efficiency to HA, and that different viral strains show different receptor specificities according to their host tropism.

### Production of synthetic sialosides

The biggest technological issue in the development of these promising therapeutic molecules is the obtention/production of these specific sialoside ligands.

Preparation of structurally diverse sialosides can be achieved through chemical synthesis, enzymatic synthesis, or modification of natural sialosides. Extraction and purification from natural sources is not considered, since only very small amounts can be obtained by this way.

Long chain sialylated polylactosamine appear to be the best candidates to efficiently prevent or treat influenza infection by a decoy strategy. However, chemical or enzymatic production of long chain sialosides is not possible at large scale. Therefore, the best alternative for production of these sialosides is the microbiological approach, i.e. production by fermentation of specific engineered microorganisms.

A method of production of 3'sialyllactose and 6'sialyllactose by fermentation has been disclosed in the international application WO 2007/101862.

Furthermore, fermentation process is already used for the industrial, large scale production of human milk oligosaccharides for the infant formula market (Bych *et al* 2019). This technology is based on the expression of specific recombinant glycosyltransferase genes in *Escherichia coli* strains, that have been metabolically engineered to overproduce the sugar nucleotide used in glycosylation reactions.

Even though the production of polylactosamine structure in metabolically engineered bacteria had been reported, the microbial production of long chain sialylated polylactosamine seems difficult to achieve, in reason of:
- the complexity of the molecular structure, and
- the number of expected side products, whose production would dramatically reduce the production yield.

It is the main object of the invention to disclose novel structures of long chain sialosides, which efficiently bind to pathogenic viruses, in particular human influenza viruses, and which can be produced in large quantity by a fermentation process.

### SUMMARY OF THE INVENTION

The present invention relates to a synthetic sialoside presenting the formula (I):

Neu5Ac-α2-6-R1(R2)[GlcNAcβ1-4]n-GlcNAc Formula (I)

Wherein:
- GlcNAc is N-acetylglucosamine;
- GlcNAcβ1-4 is a N-acetylglucosamine unit linked with a β1-4 link;
- n is superior or equal to 1;
- R1 is a glycan structure comprising at least one galactose (Gal) and where the glycan structure has a main chain comprising five monosaccharides at most; and
- R2 is chosen among the following groups: H, fucose linked with a α1-3 link (Fucα1-3) or a α1-4 link (Fucα1-4).

The present invention also concerns a multivalent sialoside comprising a support with multiple synthetic sialosides as described above.

Another object of the invention is a pharmaceutical composition comprising, in a pharmaceutically acceptable vehicle, at least one multivalent sialoside as described above.

Another object of the invention is a medical device comprising at least one multivalent sialoside as described above.

The present invention also concerns a multivalent sialoside of the invention, or a pharmaceutical composition of the invention, for its use as a medicament.

Furthermore, the present invention relates to a multivalent sialoside of the invention, or a pharmaceutical composition of the invention, for its use in the prevention and/or the treatment of an infection due to a virus having an affinity for sialic acid. Said virus is in particular an influenza virus, preferably a human, equin, porcine or avian influenza virus.

### BRIEF DESCRIPTION OF THE FIGURES

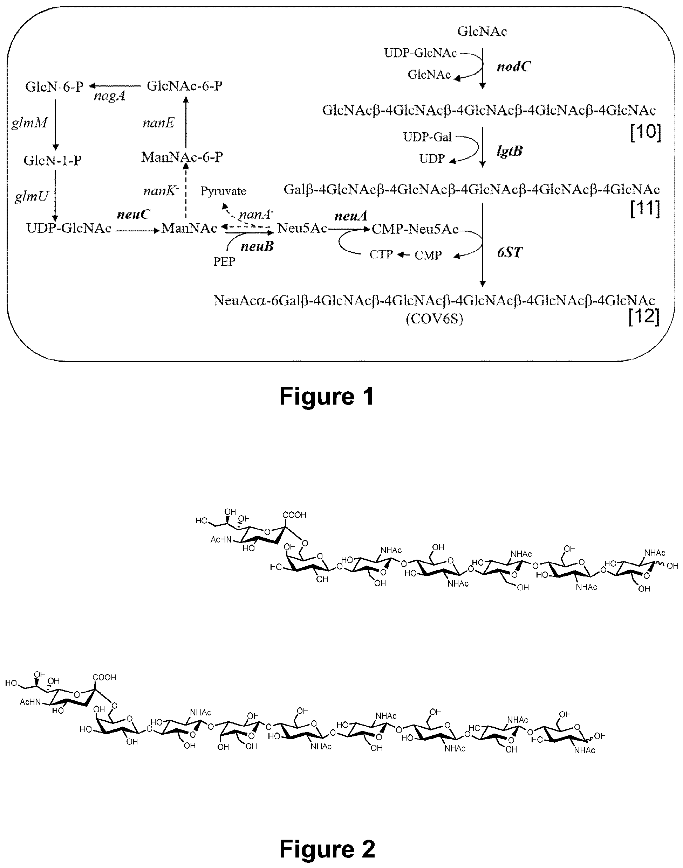

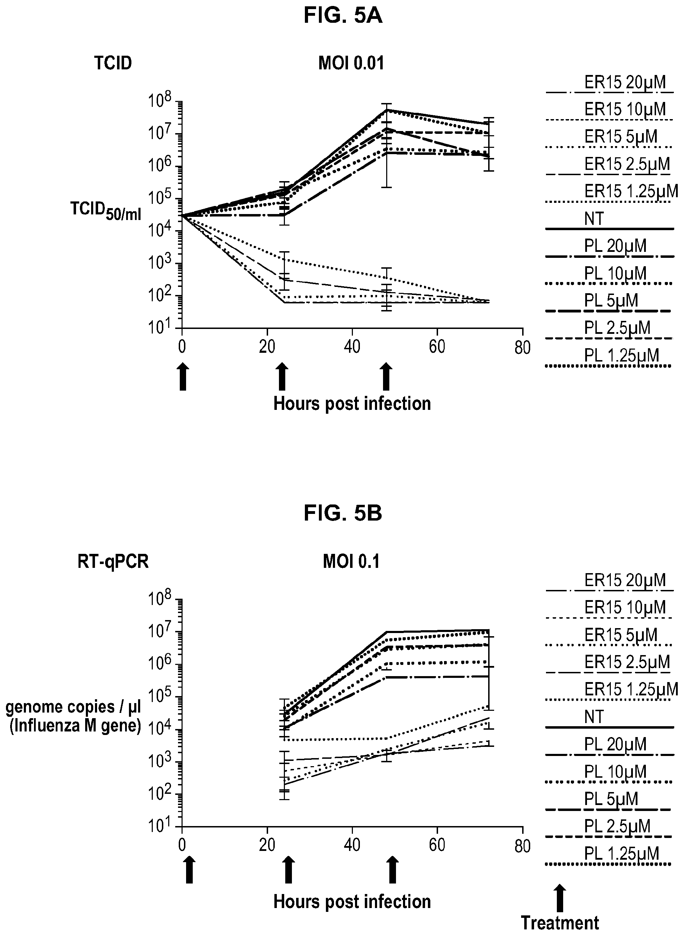

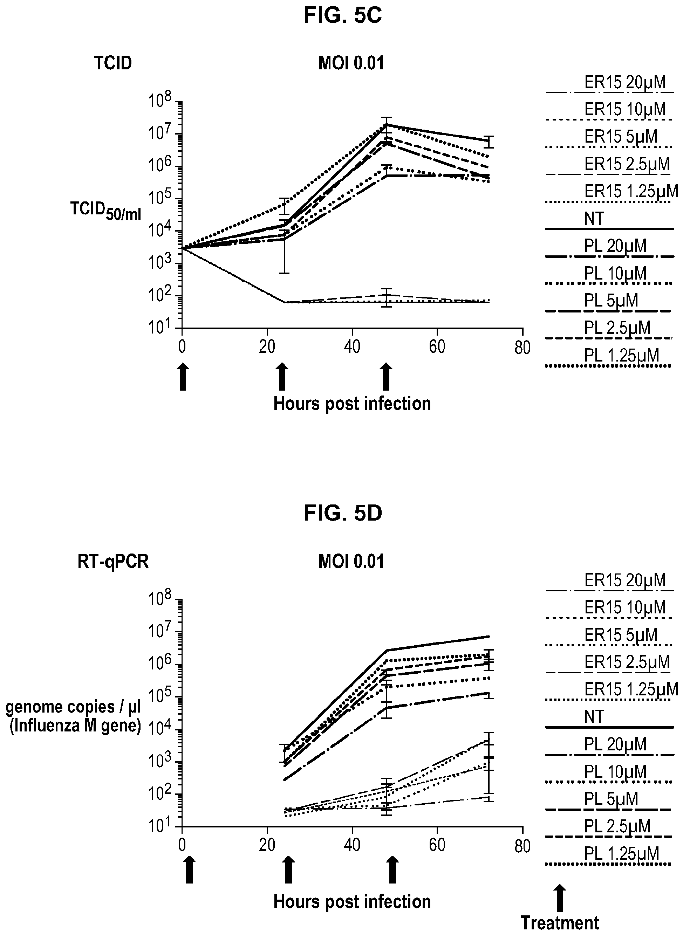

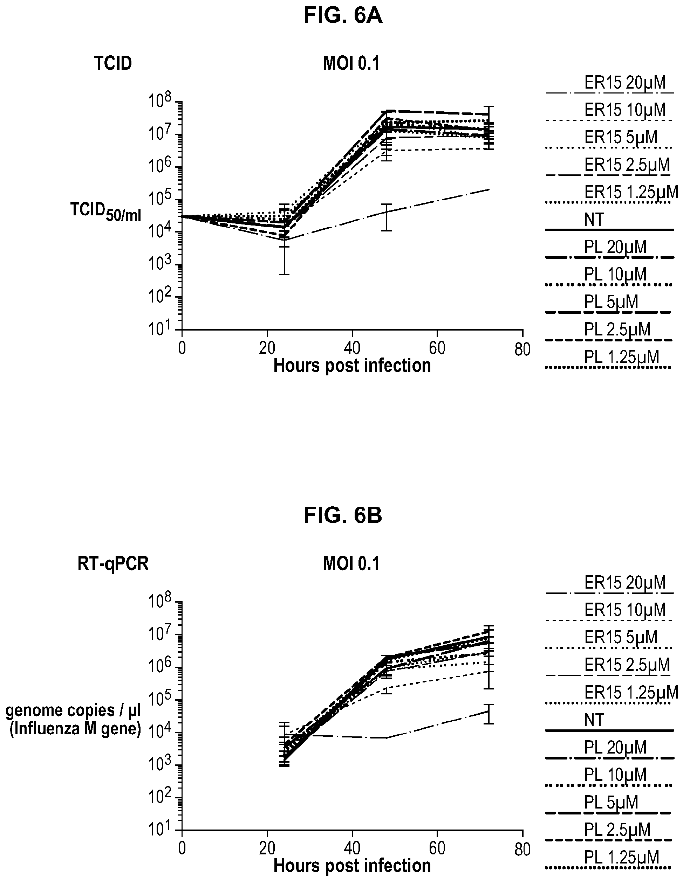

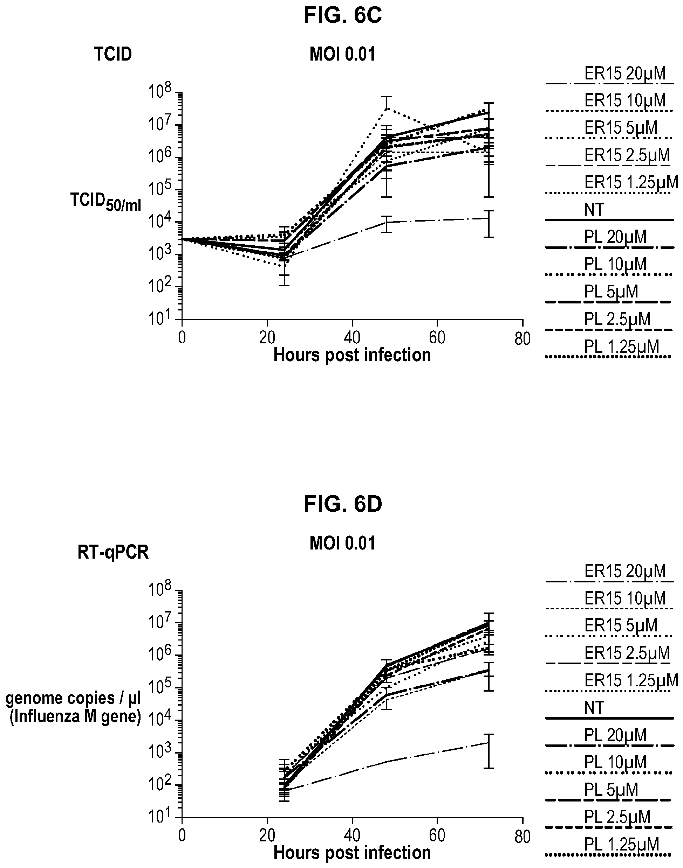

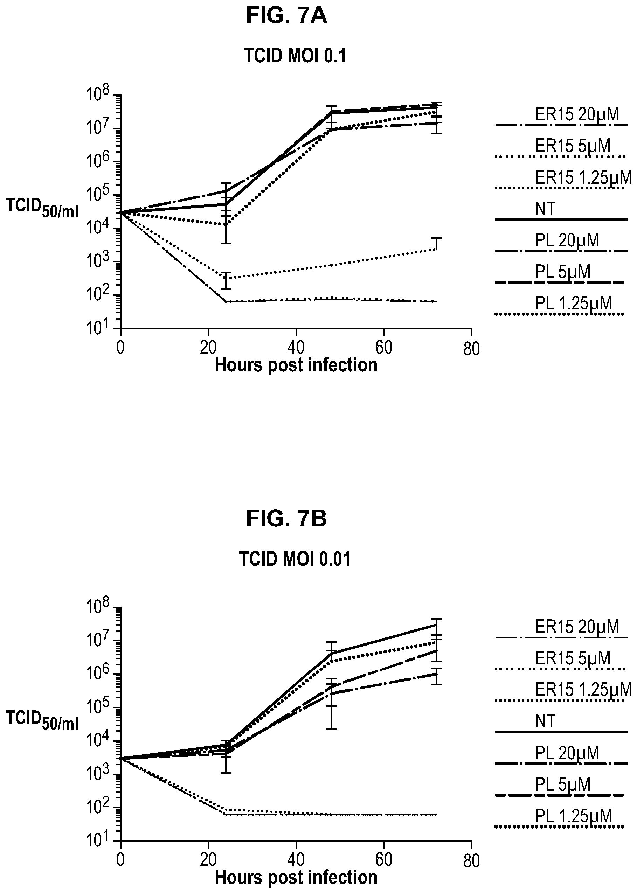

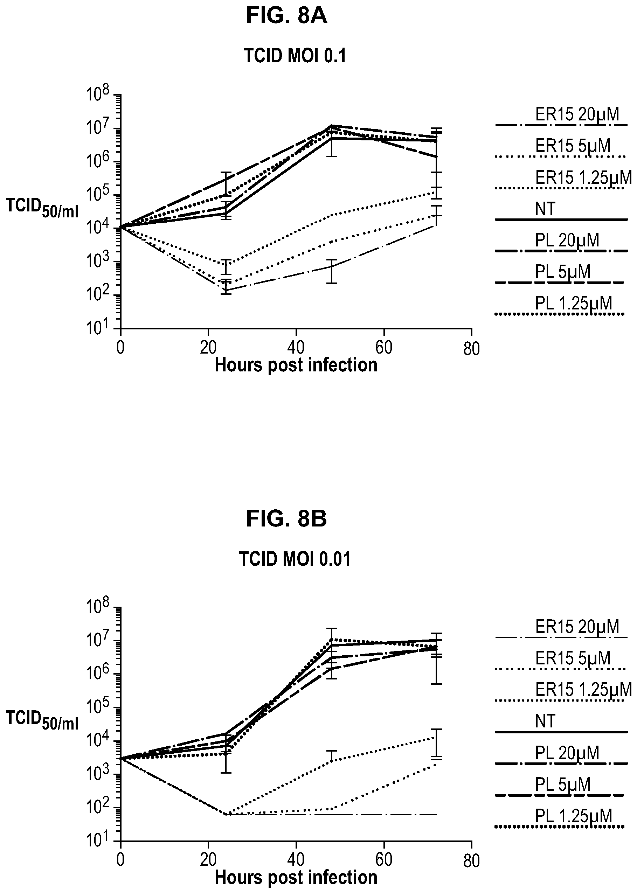

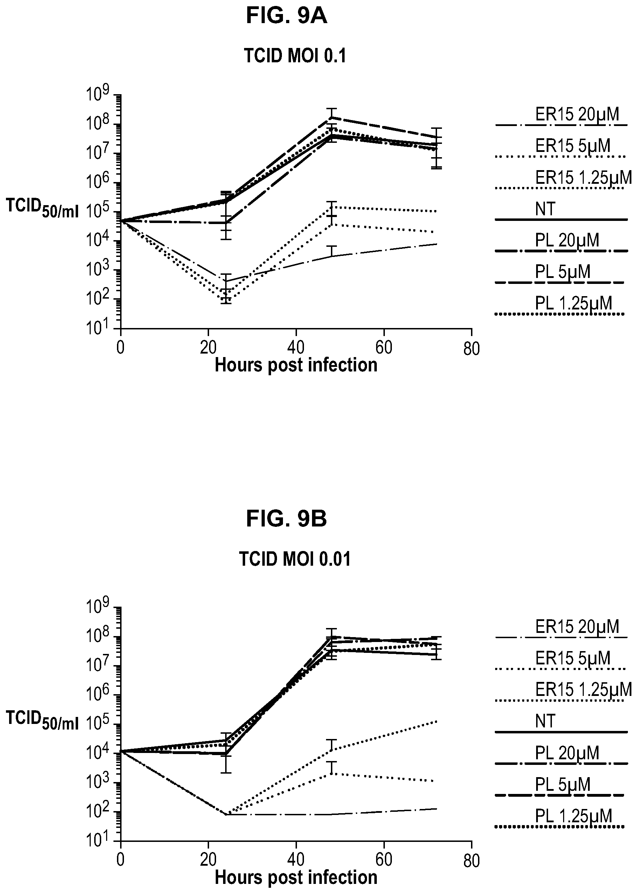

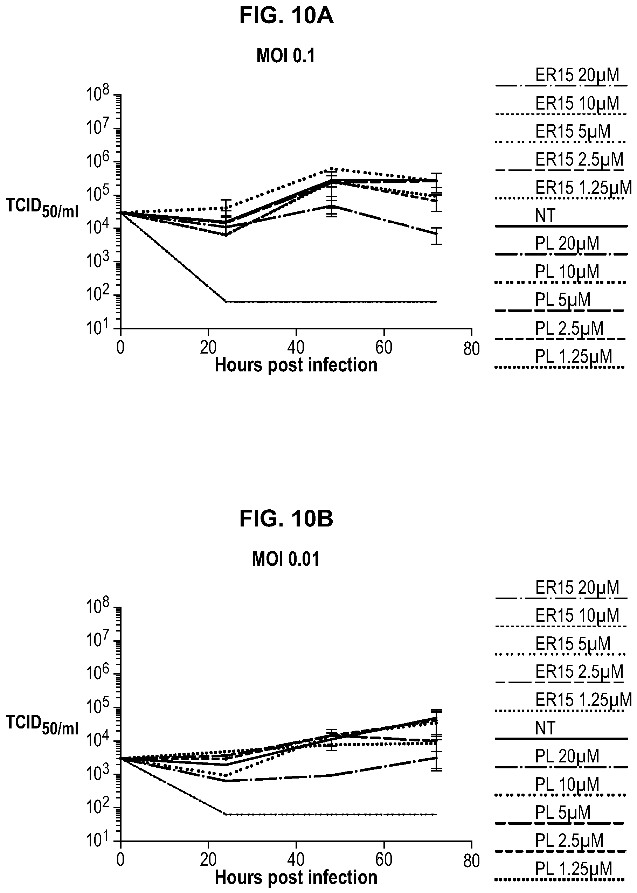

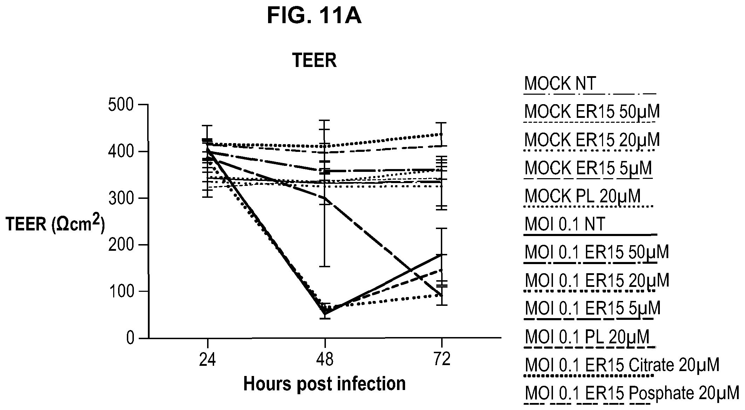

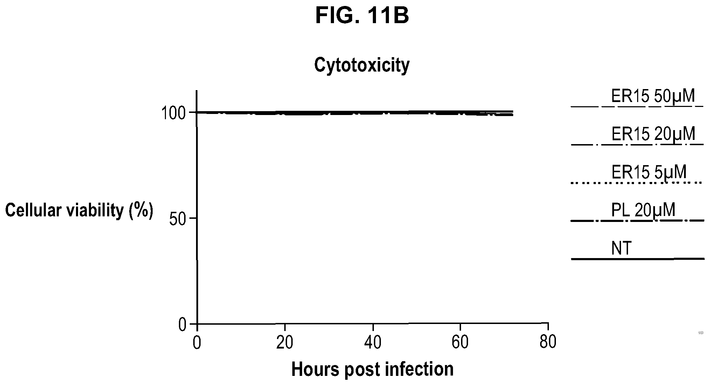

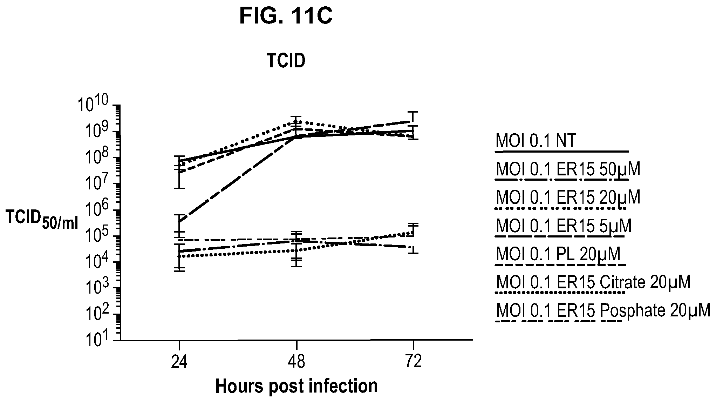

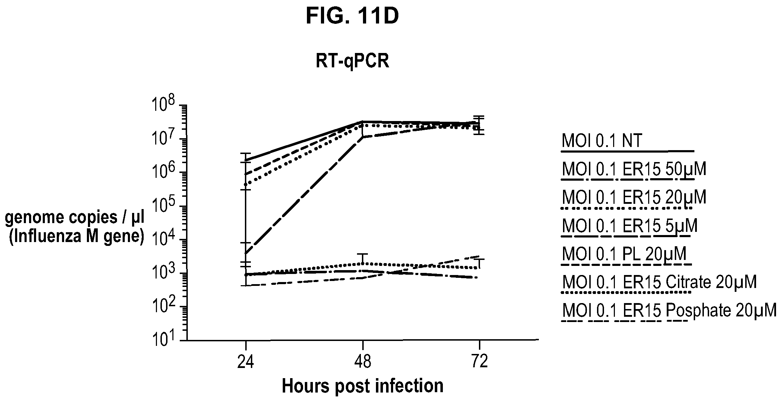

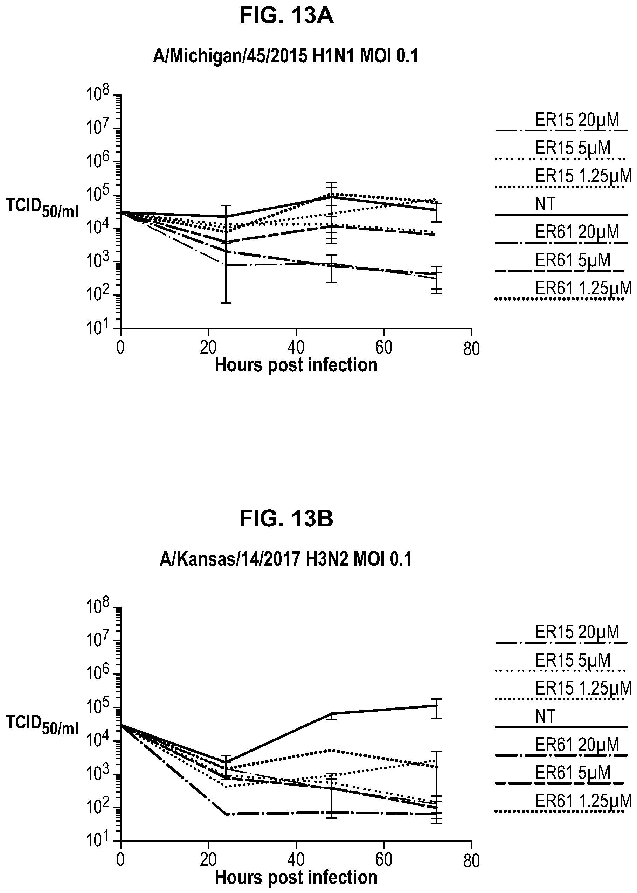

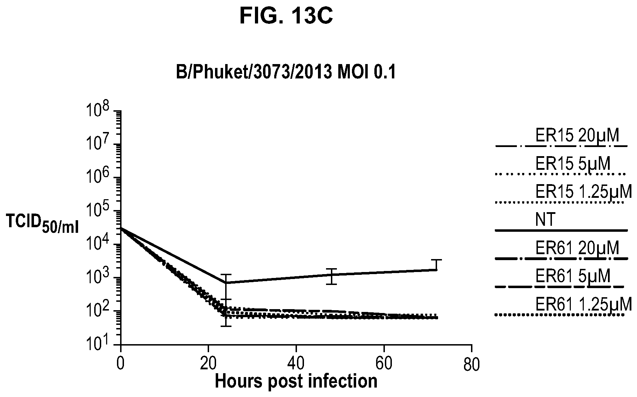

**Figure 1****. Genetically engineered metabolic pathway for COV6S biosynthesis with SCH6 strain.**
   [10] chitopentaose
   [11] intermediate hexasaccharide
   [12] sialylated heptasaccharide COV6S
   It has been shown that chitooligosaccharide such as chitopentaose [10] could be used as acceptor by β1-4 galactosyltransferase to form an hexasaccharide [11] containing a terminal LacNAc motif at its non-reducing end. Sialylation of [11] to form the sialylated heptasaccharide COV6S [12] is then possible with the additional expression of an α2-6 sialyltransferase in a metabolically engineered *E. coli* strain. Since GlcNAC is not a substrate for α2-6 sialyltransferase, formation of side products is prevented, and COV6S [12] is almost the only end product.
**Figure 2****. Developed structures of two sialosides of the invention**
   COV6S of formula (II) is presented on top; an example of another sialoside structure (formula (I) with R1 = Galβ1-4GlcNAcβ1-3Galβ1-4, R2 = H, n =4) is presented on bottom.
**Figure 3****. Schematic representation of the Hemagglutination inhibition assay (HI or HAI)**
**Figure 4****. Schematic representation of the microneutralisation assay (MN)**
**Figure 5****. Potential therapeutic effects of the ER15 compound are assayed with three post-infection treatments** at **D0 (1 hour pi), D1 and D2.**
   The A/California/7/09 H1N1 virus is incubated for 1 hour at 37°C on the cells. ER15 is used at 5 different concentrations (20; 10; 5; 2.5 and 1.25µM) vs untreated conditions (NT: untreated and PL: delivery of increasing amounts of polylysine). Supernatant samples are collected at D1, D2 and D3 post-infection to quantify viral production by (i) titration of the number of infectious particles per ml (TCID50/ml) on MDCK cells and (ii) quantification of the viral genome (RT-qPCR).
   A and C: Measure over the time (hours post infection: hpi) of generated infectious particles, expressed as TCID50/ml;
   B and D: Measure over the time (hours post infection: hpi) of viral mRNA by RT-qPCR;
   A and B: Multiplicity of infection (MOI) = 0.1
   C and D: Multiplicity of infection (MOI) = 0.01
**Figure 6****. Potential therapeutic effects of the ER15 compound are assayed with three post-infection treatments at D0 (1 hour pi), D1 and D2**
   Experimental conditions are the same than for figure 5, except that the A/Texas/50/2012 H3N2 virus is incubated for 1 hour at 37°C on the cells.
   A and C: Measure over the time (hours post infection: hpi) of generated infectious particles, expressed as TCID50/ml;
   B and D: Measure over the time (hours post infection: hpi) of viral mRNA by RT-qPCR;
   A and B: Multiplicity of infection (MOI) = 0.1
   C and D: Multiplicity of infection (MOI) = 0.01
**Figure 7****. Potential therapeutic effects of the ER15 compound against A/California/7/09 H1N1 strain are assayed with a single post-infection treatment at D0 (1 hour pi)**
   A: Multiplicity of infection (MOI) = 0.1
   B: Multiplicity of infection (MOI) = 0.01
**Figure 8****. Therapeutic properties of ER15 against A/California/7/09 H1N1 strain, with only one administration concomitant with the viral infection**
   A: Multiplicity of infection (MOI) = 0.1
   B: Multiplicity of infection (MOI) = 0.01
**Figure 9****. Prophylactic properties of ER15 against A/California/7/09 H1N1 strain, with a single treatment carried out one day before infection**
   A: Multiplicity of infection (MOI) = 0.1
   B: Multiplicity of infection (MOI) = 0.01
**Figure 10****. Therapeutic properties of ER15 against H1N1 A/Lyon/969/09 strain** Experimental conditions are the same than for figure 5, except that the A/Lyon/969/09 H1N1 virus is incubated for 1 hour at 37°C on the cells.
   A: Multiplicity of infection (MOI) = 0.1
   B: Multiplicity of infection (MOI) = 0.01
**Figure 11****. ER15 efficacity tested on infected *in vitro* epithelium models and evaluation of cytotoxicity**
   Epithelia are infected with the A/California/7/2009 H1N1 strain at the apical pole in 150µl at an infection multiplicity of 0.1.
   The lyophilized compound ER15 is mixed with:
      - water,
      - phosphate buffer (2g/L glycerin 9g/L NaCl 10mM pH = 7.9) and
      - citrate buffer (2g/L glycerin 9g/L NaCl 10mM pH = 6.0)
      at a concentration of 0.1mM.
   **A -** Measure of trans-epithelial electrical resistance (TEER) daily
   A negative control is "mock NT" cells: uninfected, untreated cells.
   Another negative control is "PL": treatment of cells with polylysine diluted in water at 0.1mM.
   **B-** Quantification of the release of lactate dehydrogenase (LDH) of mock (uninfected) cells treated with ER15, polylysine (PL) or untreated (NT). Samples are collected daily from the basal medium.
   **C-** Quantification of viral production, by titration of generated infectious particles per ml (TCID50/ml). Samples are collected at the apical pole at D1, D2 and D3.
   **D-** The same samples are submitted to a quantification of the viral genome by RT-qPCR.

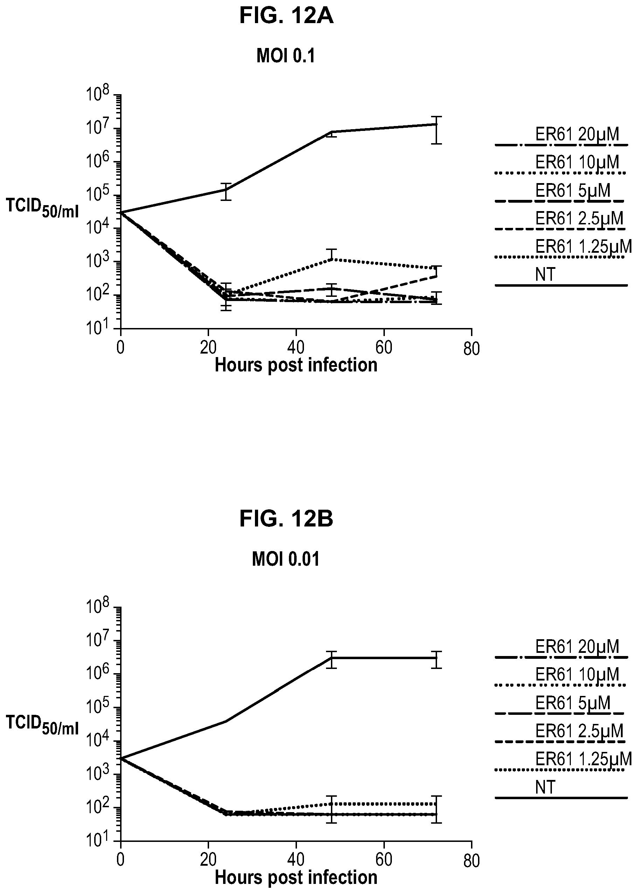

**Figure 12****. Therapeutic properties of ER61 against H1N1 A/California/7/09 strain** Experimental conditions are the same than for figure 5, except that the ER61 compound is used. Measure over the time (hours post infection: hpi) of generated infectious particles, expressed as TCID50/ml
   A: Multiplicity of infection (MOI) = 0.1
   B: Multiplicity of infection (MOI) = 0.01
**Figure 13****. Therapeutic effects of the ER15 and ER61 compounds against recent strains.**
   The tested virus strain is incubated for 1 hour at 37° C on the A549 cells, at MOI 0.1. ER15 and ER61 are used at 3 different concentrations (20; 5; and 1.25 µM) vs untreated conditions (NT: untreated). Three post-infection treatments at D0 (1 hour pi), D1 and D2 are performed. Supernatant samples are collected at D1, D2 and D3 post-infection to quantify viral production by titration of the number of infectious particles per ml (TCID50/ml).
   Tested virus strains are the following:
      A) A/Michigan/45/2015 H1N1
      B) A/Kansas/14/2017 H3N2.
      C) B/Phuket/3073/2013.

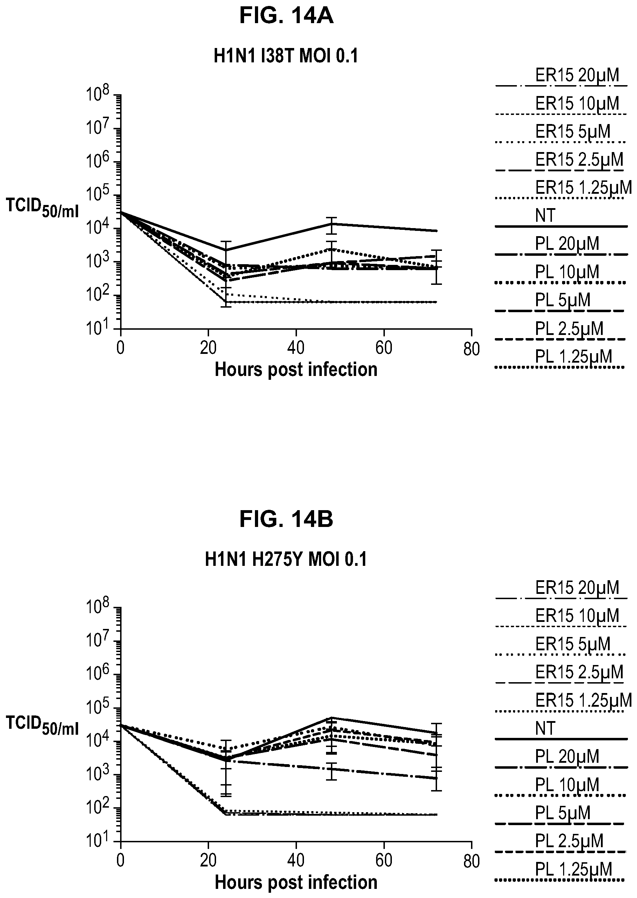

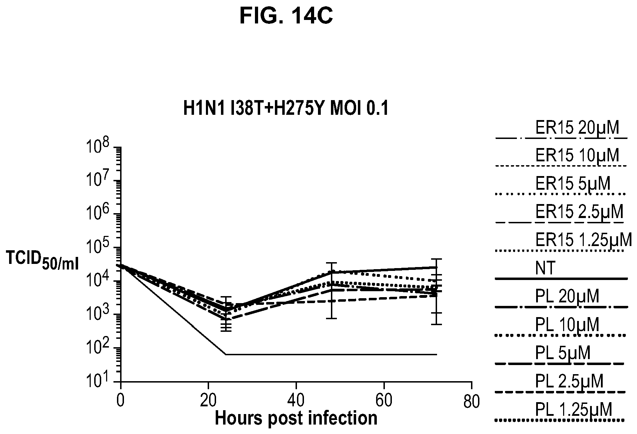

**Figure 14****. Therapeutic effects of the ER15 compound against resistant H1N1 virus strains.** Experimental conditions are the same than those of Figure 13. Three post-infection treatments are performed with ER15 at D0 (1 hour pi), D1 and D2 on infected cells with the following viral strains obtained by reverse genetic:
   A) RG H1N1 A/Lyon/969/09 I38T
   B) RG H1N1 A/Lyon/969/09 H275Y
   C) RG H1N1 A/Lyon/969/09 I38T + H275Y

### DETAILED DESCRIPTION OF EMBODIMENTS OF THE INVENTION

The present invention concerns a synthetic sialoside presenting the formula (I):

Neu5Ac-α2-6-R1(R2)[GlcNAcβ1-4]n-GlcNAc Formula (I)

Wherein:
- GlcNAc is N-acetylglucosamine;
- GlcNAcβ1-4 is a N-acetylglucosamine unit linked with a β1-4 link;
- n is superior or equal to 1, in particular n is comprised between 1 and 4;
- R1 is a glycan structure comprising at least one galactose (Gal) and where the glycan structure has a main chain comprising five monosaccharides at most; and
- R2 is chosen among the following groups: H, fucose linked with a α1-3 link (Fucα1-3) or a α1-4 link (Fucα1-4).

Unless stated otherwise, the following terms and phrases as used herein are intended to have the following meanings.

In the sense of the invention, the term sialoside designates a sialic acid conjugate, consisting of a sialic acid bound to a carbohydrate by an O-glycosidic bond.

Sialic acids are a family of monosaccharides that comprises 43 naturally occurring derivatives of 5-amino-3,5-dideoxy-D-glycero-D-galacto-non-2-ulosonic acid (neuraminic acid), a saccharide composed of a nine-carbon backbone, that is ubiquitously expressed in all vertebrates. The most common sialic acid is 5-acetamido-2-keto-3,5-dideoxy-D-glycero-D-galactonononic acid, also named N-acetylneuraminic acid or sialic acid, designated with the abbreviation Neu5Ac.

Sialic acids are recognized as a key component of receptors for bacterial toxins and a range of viruses.

The term "synthetic" emphasizes the artificial nature of the sialoside according to the invention. Indeed, this sialoside is not found in nature and its structure has been designed by the inventors. Advantageously, this synthetic sialoside has been designed in order to present the best affinity with surface proteins of viruses having an affinity for sialic acids, in particular with hemagglutinins of the influenza virus strains. Each sialoside of the invention may have a different affinity for various hemagglutinins, in particular each sialoside of the invention may have a preferential binding to one specific hemagglutinin.

Other terms and abbreviations are well known by the man skilled in the art, in particular:
- N-acetylglucosamine (GlcNAc) is an amide derivative of glucose. It is a secondary amide between glucosamine and acetic acid. This molecule is widely express edamong the living organisms, since it is part of biopolymers constituting cell walls. Its CAS number is 7512-17-6.
- β1-4 link designates a covalent glycosidic bond of beta type that joins a carbohydrate group to another group.

α- and β-glycosidic bonds are distinguished by the relative stereochemistry of the anomeric position and the stereocenter furthest from C1 in the saccharide. An α-glycosidic bond is formed when both carbons have the same stereochemistry, whereas a β-glycosidic bond occurs when the two carbons have different stereochemistry.
- a "glycan structure" is a carbohydrate structure consisting of glycosidically linked monosaccharides forming a "chain".
- Galactose (Gal) is an aldohexose, an epimer of glucose. Its CAS number is 59-23-4.
- H designates a hydrogen atom.
- Fucose (Fuc) is a hexose deoxy sugar, also designated as 6-deoxy-L-galactose. Its CAS number is 2438-80-4. It may be bound with a α1-3 link (Fucα1-3) or a α1-4 link (Fucα1-4).

Formula (I) corresponds to a novel long chain sialoside structure designed by the inventors, comprising at least one N-acetylglucosamine unit linked with a β1-4 link, i.e. n is superior or equal to 1. In some embodiments, n is equal to 2, 3, 4, 5, 6, 7, 8 or more. In a preferred embodiment, n is comprised between 1 and 4.

In formula (I), the component R1 is defined as a glycan structure comprising at least one galactose (Gal). R1 is composed of a chain of one or more monosaccharides that are linked in a row, which defines the "main chain" of R1. On this main chain, monosaccharides or secondary chains comprising at least two monosaccharides may be grafted.

R1 is a glycan structure that may be chemically modified, in particular by addition of one or more methyl groups ("methylation") or of one or more sulfate (SO₃) groups ("sulfation") on one or more monosaccharides constituting the glycan structure, in particular on monosaccharides of the main chain.

In a particular embodiment of the invention, R1 is a glycan structure with a main chain comprising five monosaccharides at most. In this embodiment, R1 is composed of one, two, three, four or five linked monosaccharides, forming one main glycan chain. This main glycan chain may be further grafted with secondary glycan chains, branched on one or more monosaccharides of the main chain.

Preferentially, R1 is composed of a main chain comprising one, two or three linked monosaccharides, forming one main glycan chain. This main glycan chain may be further grafted with secondary glycan chains, branched on one or more monosaccharides of the main chain.

In a specific embodiment, R1 is composed of a main chain comprising three linked monosaccharides.

In another specific embodiment, R1 is an ungrafted main chain consisting in three monosaccharides.

In another specific embodiment, R1 is a grafted main chain consisting in three monosaccharides.

In another specific embodiment, R1 is composed of a single monosaccharide that is galactose.

In another specific embodiment of the invention, R1 is a glycan structure where the at least one galactose is linked at one of the extremities of the main chain.

In a first configuration, R1 comprises one galactose at one of the extremities of the main glycan chain. In a second configuration, R1 comprises two galactoses, each one being linked at each of the extremities of the main glycan chain.

In a specific embodiment of the invention, R1 is chosen among the following groups:
- Galβ1-4,
- Galβ1-3,
- Galβ1-4GlcNAcβ1-3Galβ1-4, and
- Galβ1-4 (Fucα1-3)GlcNAcβ1-3Galβ1-4.

In another specific embodiment of the invention, R2 is H. According to this embodiment, the structures having the formula (III) as presented below are an object of the invention:

Neu5Ac-α2-6-R1-[GlcNAcβ1-4]n-GlcNAc (Formula III)

Wherein:
- n is superior or equal to 1, in particular n is comprised between 1 and 4; and
- R1 is a glycan structure comprising at least one galactose (Gal).

A non-exhaustive list of sialoside structures according to the invention is presented below:
- Neu5Acα2-6Galβ1-4[GlcNAcβ1-4]ₙ-GlcNAc
- Neu5Acα2-6Galβ1-4(Fuca1-3)[GlcNAcβ1-4]ₙ-GlcNAc
- Neu5Acα2-6Galβ1-3[GlcNAcβ1-4]ₙ-GlcNAc
- Neu5Acα2-6Galβ1-3(Fucα1-4)[GlcNAcβ1-4]ₙ-GlcNAc
- Neu5Ac-α2-6Galβ1-4GlcNAcβ1-3Galβ1-4[GlcNAcβ1-4]ₙ-GlcNAc
- Neu5Ac-α2-6Galβ1-4(Fucα1-3)GlcNAcβ1-3Galβ1-4[GlcNAcβ1-4]ₙ-GlcNAc
- Neu5Ac-α2-6Galβ1-4GlcNAcβ1-3Galβ1-4(Fucα1-3)[GlcNAcβ1-4]ₙ-GlcNAc
- Neu5Ac-α2-6Galβ1-4(Fucα1-3)GlcNAcβ1-3Galβ1-4(Fucα1-3)[GlcNAcβ1-4]ₙ-GlcNAc

According to a specific embodiment of the invention, R1 is a galactose linked with a β1-4 link (Galβ1-4). In this embodiment, the synthetic sialoside presents the following formula (II):
Neu5Acα2-6Galβ1-4GlcNAcβ1-4GlcNAcβ1-4GlcNAcβ1-4GlcNAcβ1-4GlcNAc

This specific sialoside of formula (II) is also designated in the present application, in particular in the examples section, as "COV6S". Its developed structure is represented in the top of figure 2.

As shown in the examples, other structures of sialosides have been synthetized and tested. These structures corresponding to the general formulas (I) and (III) are presented in the following table 1.

**Table 1. Sialosides presented in the examples section**

| Sialoside name | Structure of sialoside | R1 is | R2 is | n = |
|---|---|---|---|---|
| 6SL | NeuAcα2-6Galβ-4Glc | Not included in formula (I) | | |
| LSTc | Neu5Aca2-6Galβ1-4GlcNAcβ1-3Galβ1-4Glc | Not included in formula (I) | | |
| COIII6S | Neu5Acα2-6Galβ1-4GlcNAcβ1-4GlcNAcβ1-4GlcNAc | Galβ1-4 | H | 2 |
| COIV6S | Neu5Acα2-6Galβ1-4GlcNAcβ1-4GlcNAcβ1-4GlcNAcβ1-4GlcNAc | Galβ1-4 | H | 3 |
| 1-COV6S | Neu5Acα2-6Galβ1-3GlcNAcβ1-4GlcNAcβ1-4GlcNAcβ1-4GlcNAcβ1-4GlcNAc | Galβ1-3 | H | 4 |
| COV6S | Neu5Acα2-6Galβ1-4GlcNAcβ1-4GlcNAcβ1-4GlcNAcβ1-4GlcNAcβ1-4GlcNAc | Galβ1-4 | H | 4 |

These sialosides COIII6S, COIV6S, 1-COV6S et COV6S are representative of the synthetic silaosides according to the invention, which contain a chitooligosaccharide terminal chain. These novel sialosides can be used to construct multivalent sialoside compounds which can efficiently bind to influenza virus, and be used as drug against influenza infection.

Each sialoside may present a distinct specificity for hemagglutinins. Indeed, according to their structure, sialosides may present a specificity of binding to hemagglutinins of various strains of virus.

### Multivalent sialoside

The present invention also concerns a multivalent sialoside comprising a support with multiple synthetic sialosides.

In the sense of the invention a "multivalent sialoside" designates a molecular structure comprising multiple sialosides, said structure showing an increased binding affinity with its receptors (such as hemagglutinins) in comparison with the binding affinity of a single sialoside.

The initial virus attachment to the host cell is regulated by multivalent interactions, where multiple HA trimers are involved in the interaction with multiple sialoside ligands expressed on cell surface glycoproteins and glycolipids. The binding of soluble monomeric sialoside is too weak to competitively disrupt these strong polyvalent interactions. According to the theory of the "multivalent effect", a rational drug design based on the inhibition of virus/host cell binding must include the synthesis of multivalent sialoside compounds.

In the case of inhibition of influenza virus, it has been shown that "multivalent sialosides" ligands have a stronger affinity for hemagglutinins, and therefore are more appropriate for acting as antagonists, by blocking the HA binding sites and therefore inhibiting their interaction with glycoproteins present on the surface of host cells. This process of saturation of hemagglutinin binding sites is based on multivalent binding, to gain stability and prevent dissociation of viruses.

Many different strategies have been developed to produce various kind of multivalent sialoside structures. A variety of synthetic multivalent sialoside inhibitors of influenza virus adhesion has been already developed. They are based on supports such as liposomes (Kingery-Wood *et al* 1992), dendrimers (Reuter *et al* 1999), synthetic polymers (Gambaryan *et al* 2005), chitosan (Umemura *et al.,* 2010) polysaccharides (Li *et al* 2011) and gold nanoparticules (Papp *et al* 2010).

As used herein, the phrases "support with multiple sialosides", "support bearing multiple sialosides", "multiple sialodises grafted on a support", "support carrying sialosides" and "multiple sialosides loaded on support" are equivalent and indifferently used; they all refer to a multivalent sialoside according to the invention, comprising multiple chains of sialosides bound, covalently or non-covalently, to any support known by the man skilled in the art.

Said multiple sialosides are usually all identical; nevertheless, multivalent sialosides comprising at least two, three or four different sialosides bound on a single support may also be generated.

In a specific embodiment of the invention, the support consists of liposomes, polymer(s), dendrimers, or nanoparticules.

Liposomes can be used to encapsulate drugs, proteins, genes, and fluorescent dye molecules for applications in imaging and controlled drug release. Liposomes carrying sialosides can be prepared from sialosidic phospholipids and amphiphilic precursors. Different sizes of liposomes can be generated.

The use of polymer, generating a polymeric network of sialosides, is one of the earliest known examples of multivalent sialosides. The main approaches that have been developed to obtain sialosides bearing-polymers are:
- the assembly of monomeric sialoside ligands by using an acrylate free-radical polymerization method; and
- the coupling of sialoside ligands on commercially available, well-defined polymer scaffolds.

Dendrimers are important multivalent scaffolds that are used to decorate ligands of interest in a distinct homogeneous symmetrical form. In comparison to polymers, dendrimers offer more-controllable monodispersity and persistent shapes. Moreover, the 3D geometry of sialosides carrying-dendrimers closely mimics the decoration of silaoside groups over cell surfaces.

Nanoparticles are well-organized, robust dendrimers that exhibit large surface areas and have inherent optical, electrochemical, and magnetic properties that facilitate the sensing and imaging of specific interactions. Several types of sialosides bearing-nanoparticles have been reported in the literature, the most prevalent of which are gold, silver, silica, iron, cadmium, selenium, and virus-like nanoparticles.

In a specific embodiment of the invention, the support is a polymer, preferentially a natural polymer, and in particular consists in polylysine (ε-Poly-L-lysine).

ε-Poly-L-lysine (ε-PL) is a homopolymer linked by the peptide bond between the carboxylic and the epsilon amino group of adjacent lysine molecules. It is naturally occurring, water soluble, biodegradable, edible and nontoxic towards humans and environment. ε-PL consists of 25-35 L-lysine residues. It is industrially produced by aerobic fermentation using *Streptomyces albulus.* It has antimicrobial activity and has been approved as a food preservative in Japan and in US.

The multivalent sialoside is structurally characterized by its constituents (support and sialosides) and also by its grafting rate.

The grafting rate, also designated as the coupling yield, is defined as the percentage of reacting groups of the support which are substituted with a sialoside.

The man skilled in the art knows how to modulate the grafting rate of sialosides onto their support. In particular, the grafting rate is controlled by the reaction conditions, notably by the proportional quantities of support and sialosides, as is shown in example 6.

The grafting rate usually varies from 10 % to 100 %.

In a specific embodiment of the invention, the multivalent sialoside presents a grafting rate comprised between 20% and 100%.

Preferentially, the grafting rate is comprised between 30% and 100%, 40% and 100%, 50% and 100%, 60% and 100%, 70% and 100%, 80% and 100%, or is comprised between 90% and 100%.

### Pharmaceutical composition

The present invention also concerns a pharmaceutical composition comprising, in a pharmaceutically acceptable vehicle, at least one multivalent sialoside as described above.

The present invention also concerns a veterinary composition comprising, in a pharmaceutically acceptable vehicle, at least one multivalent sialoside as described above. Advantageously, said multivalent sialoside is designed for the inhibition of a virus having animal host cells.

This pharmaceutical or veterinary composition comprises an effective amount of at least one multivalent sialoside according to the invention.

For the purposes of the invention, "effective amount" means an amount of multivalent sialoside sufficient to inhibit the proliferation and/or replication of the virus, and/or the development of the viral infection within the infected organism. This inhibition can be quantified, for example, by measuring viral replication.

For example, *in vitro,* the so-called "effective" amounts are comprised between 1 µm and 1 mM, as shown in examples 9 to 11.

The pharmaceutical or veterinary composition according to the invention may comprise one, two, three, four, five or more multivalent sialosides. Advantageously, each multivalent sialoside in the composition presents a distinct specificity for a specific viral strain.

According to the invention, the term "pharmaceutically acceptable vehicle" refers to one or more pharmaceutically acceptable vehicles or excipients whose administration to an individual or an animal is not accompanied by significant deleterious effects, and which are well known to the person skilled in the art.

The pharmaceutical or veterinary compositions according to the present invention are suitable for oral, sublingual, inhalation, subcutaneous, intramuscular, intravenous, transdermal, ocular or rectal administration.

According to a preferred aspect of the invention, the pharmaceutical composition is characterized in that it is in a galenic form adapted for administration by inhalation.

Inhalation refers to absorption through the respiratory tract. It is in particular a method of absorption of compounds for therapeutic purposes, of certain substances in the form of gas, microdroplets or powder in suspension.

The administration of pharmaceutical compositions by inhalation, i.e. through the nasal and/or oral passages, is well known to the skilled person.

There are two types of administration by inhalation:
- administration by insufflation when the compositions are in the form of powders, and
- administration by 15ebulization when the compositions are in the form of aerosols (suspensions) or in the form of solutions, e.g. aqueous solutions, under pressure. The use of a nebulizer or a sprayer will then be recommended to administer the pharmaceutical or veterinary composition.

The galenic form considered here is therefore chosen from: a powder, an aqueous suspension of droplets, or a solution under pressure. Administration by nasal droplets is preferred.

### Medical device

The present invention also concerns a medical device comprising at least one multivalent sialoside as described above.

In an embodiment, said medical device consists of a support coated with at least one multivalent sialoside of the invention.

In a specific embodiment, the support is coated with multiple (at least two) multivalent sialosides, each multivalent sialoside having a specific affinity for hemagglutinins expressed at the surface of different viral strains.

For example, the support is coated with three multivalent sialosides, one being specific of H1N1 influenza virus strain, the second one being specific of H2N2 influenza virus strain and the third one being specific of H3N2 influenza virus strain.

Said support may be, for example, a respiratory protection mask or a filter to be inserted into a mask. Protection masks are increasingly requested by the public, following the appearance of major pandemics. Said support may also be gloves or any other personal protection device.

Said support is coated with, for example, a multivalent sialoside consisting of a polymer grafted with multiple sialosides according to the invention. Each sialoside may present a distinct specificity for different hemagglutinins.

Advantageously, this medical device can be used to trap and hold at least one virus having an affinity for sialic acid, in particular a human influenza virus, or multiple strains of human influenza viruses.

In another embodiment of the invention, the medical device is a nose spray comprising, as injected solution, a solution comprising at least one multivalent sialoside according to the invention.

### Medicaments and therapeutic uses

The present invention also relates to a multivalent sialoside as described, or to a pharmaceutical composition containing at least one multivalent sialoside, for its use as a medicament.

The present invention also concerns a multivalent sialoside as described, or a pharmaceutical composition containing at least one multivalent sialoside, for its use in the prevention and/or the treatment of an infection due to a virus having an affinity for sialic acid.

The present invention also concerns a multivalent sialoside as described, or a pharmaceutical composition containing at least one multivalent sialoside, for its use in the prevention of viral transmission from one host organism to another.

The phrase "a virus having an affinity for sialic acid" designates any virus, having a pathogenic action i.e. responsible of a disease in living organisms, expressing receptors to sialic acid, and therefore being able to bind to sialic acid conjugates.

Such viruses are presented in a non-exhaustive manner in the review of Matrosovich M et al., 2015. In particular, such viruses having an affinity for sialic acid are chosen among Orthomyxoviridae (including influenza viruses), Corinaviridae, Paramyxoviridae, Caliciviridae, Picornaviridae, Reoviridae, Polyomaviridae, Adenoviridae and Parvoviridae.

In a specific embodiment of the invention, this virus is an influenza virus, in particular a human, equin, porcine or avian influenza virus.

Influenza viruses are responsible for the flu disease. They are divided into three types: A, B and C.

Influenza viruses are also defined by the type of proteins on their surface. There are various influenza A virus subtypes according to the nature of the hemagglutinin (HA) and neuraminidase (NA) glycoproteins expressed on their surface: 16 types of HA and 9 types of NA have been identified in circulating influenza A viruses.

In humans, the most common influenza A viruses are from the subtypes H1N1, H2N2 and H3N2, with occasional interspecies transmission, notably from animals to human beings, of avian viruses H5N1, H7N7, H7N9, H5N2 and H9N2.

According to a particular embodiment, the influenza virus is a type A human virus selected from the H1N1, H2N2, H3N2, H5N1, H7N7, H7N9, H5N2 and H9N2 subtypes.

In another specific embodiment of the invention, the influenza virus is human, and is chosen among strains H1N1, H3N2 and B.

In the sense of the invention, the term "influenza virus" includes all types (A or B) and subtypes (H1N1, N3N2, etc) of influenza viruses. This term includes wild-type influenza strains, and mutated influenza strains, in particular strains with genetic mutations that lead to a resistance of said strains to antiviral compounds.

The compositions according to the invention are in particular intended for use in the prevention of infection with influenza viruses.

Advantageously, these compositions are intended for use in the prevention of infection with influenza mutated strains, presenting resistance to usual antiviral compounds, such as oseltamivir-resistant strains and baloxavir-resistant strains. This aspect is presented in details in example 13.

The term "prevention" refers to the fact of preventing, or at least decreasing, the probability of the appearance of an infection in a human or animal organism by at least one influenza virus. The objective is to saturate the antigenic sites of the hemagglutinins present on the surface of viruses with the multivalent sialoside of the invention, thus preventing the attachment of viruses to host cells.

The compositions according to the invention may also be intended for use in the treatment of infection with influenza viruses.

The term "treatment" refers to the fact of fighting infection with at least one influenza virus in a human or animal organism. By administering at least one composition according to the invention, the level of viral infection in the organism will gradually decrease and then completely disappear. The term "treatment" also refers to the fact of alleviating the symptoms associated with viral infection (fever, fatigue, etc.).

The present invention also relates to a combination product comprising at least one multivalent sialoside and at least one other antiviral compound, for simultaneous, separate or sequential use in the prevention and/or treatment of a viral infection by an influenza virus.

In a preferred aspect, the antiviral compound is chosen from among the classically used antiviral compounds to prevent or treat influenza. Such antiviral compounds are commercially available, and described in reference books such as Vidal. For example, Oseltamivir and Baloxavir marboxil are antiviral compounds and can be used in combination with a multivalent sialoside of the invention.

The present invention also relates to a method for preventing or treating an infection due to a virus having an affinity for sialic acid in a human being, comprising the administration to said human being of an efficient amount of at least one multivalent sialoside according to the invention.

### EXAMPLES

### Example 1. Production of the COV6S sialoside

Chitooligosaccharides are interesting alternative to polylactosamine chains for several reasons. First, both polylactosamine and chitooligosaccharide are long chain of hexose linked by beta glycosidic bond. Secondly, both structures contain N-acetylglucosamine as major component. Thirdly, chitooligosaccharides can be produced in good yield by metabolically engineered *E. coli* expressing the chittooligosaccharide synthase nodC (Samain et al 1997).

Moreover, it has been shown by Bettler et al (1999) that chitooligosaccharide such as chitopentaose [10] could be used as acceptor by β1-4 galactosyltransferase to form an hexasaccharide [11] containing a terminal LacNAc motif at its non-reducing end, as shown in Figure 1.

Sialylation of [11] to form the sialylated heptasaccharide COV6S [12] is then possible by the additional expression of an α2-6 sialyltransferase in a metabolically engineered *E. coli* strain SCH1 obtained as described below.

The strain SCH1 is constructed by transforming the host strain ZLKA with four plasmids pBSnodC, pBBR3-SS, pWKS-lgtB and pSU6ST.
a) Construction of the host strain ZLKA was described by Fierfort and Samain (2008). The strain ZLKA is a derivative of the *Escherichia coli* K12 strain DH1 (*endA1 recA1 gyrA96 thi-1 glnV44 relA1 hsdR17)* which was obtained from the Deutsche Sammlung von Mikroorganismen (reference DSM 4235) and which carry the additional mull mutation in the *lacZ lacA nanA* and *nanK* genes.
b) The pBSnodC plasmid carrying the *nodC* gene for chitinoligosaccharide synthase from *Azorhizobium caulinaudans* was constructed as described by Cottaz and Samain (2005).
c) Construction of the pBBR3-SS plasmid was described by Fierfort and Samain (2008). It carries the three genes *neuC neuB* and *neuA* from *Campylobacter jejuni* strain ATCC 4343, encoding N-acetylglucosamine-6-P epimerase, sialic acid synthase and the CMP-NeuA synthase, respectively.
d) Construction of the pWKS-lgtB plasmid carrying the galactosyltransferase *lgtB* from *Neisseria meningitidis.* This plasmid was constructed as described in Cottaz and Samain (2005).
e) Construction of the pSU6ST plasmid carrying α2-6 NeuAc transferase from *Photobacterium sp.* JT-ISH-224. This plasmid was described by Richard *et al* (2017).

This strain SCH1 is grown at high cell density as previously described (Priem et al. 2002): cultures were carried out in 3-liter reactors containing 1.5 liter of mineral culture medium, the temperature is maintained at 34°C and the pH is regulated to 6.8 with 14 % NH4OH.

The high cell density culture consisted of three phases: an exponential growth phase, which started with the inoculation of the fermenter and lasted until exhaustion of the carbon substrate (glycerol 17.5g.L-1), a 5 h fed-batch with a high glycerol feeding rate of 5 g.L-1h-1 and a 20 h fed-batch phase with a glycerol feeding rate of 3 g.L-1h-1 .

At the end of the fermentation period, bacterial cells were recovered by centrifugation (7000g, 30 min). The cell pellets were re-suspended in distilled water and the cells were permeabilized by autoclaving at 100 °C for 50 min. The mixture is centrifuged (7000g, 30 min) and the supernatant containing oligosaccharides is recovered.

The pH of the extracellular fraction is lowered to 3.0 by the addition of a strong cation exchanger resin (AmberliteIR120 H+ form). This resulted in the precipitation of proteins, which were removed by centrifugation. The pH of the clear supernatant is then adjusted to 6.0 by the addition of a weak anion exchanger (Dowex 66 free base form) and after decanting, the supernatant is loaded on a Dowex 1 (HCO3 form) column (5 x20 cm). After washing with distilled water, the acidic oligosaccharides retained on the Dowex 1 resin were eluted with a 0-500 mM continuous (NH4)₂CO3 gradient.

The eluted fractions containing COV6S were pooled and the (NH₄)₂CO₃ is removed by freeze-drying.

Interestingly the fact that GlcNAC is not a substrate for the α2-6 sialyltransferase, which prevents the formation of side products; consequently, COV6S (product [12] in figure 1) is almost the only end product.

Structure of COV6S is confirmed by nuclear magnetic resonance (NMR) and mass spectrometry.

### Example 2. Production of the type 1 COV6S sialoside

The type 1 COV6S sialoside (1-COV6S) presents the following formula:

Formula (IV) Neu5Acα2-6Galβ1-3GlcNAcβ1-4GlcNAcβ1-4GlcNAcβ1-4GlcNAcβ1-4GlcNAc

This formula corresponds to the general formula (I) with R1 = Galβ1-3, R2=H and n=4.

It is produced by fermentation of the strain SCH11, as described in example 1 for the strain SCH1.

The strain SCH11 is similar to SCH1 but the plasmid pWKS-lgtB containing the b1-3 galactosyltransferase gene is omitted, and pBBR3-SS is replaced by pBBR3-SS-β-3GalT which contains the additional gene for β1-3 Galactosyltransferase.

The plasmid pBBR3 is constructed as follows: a 1.34 kb DNA containing the sequence of a β1-3 galactosyltransferase gene is amplified by PCR using the genomic DNA of *Helicobacter pylori* ATCC43504 a template.

A Sall site is added to the left primer:
5'GGTCGACGGTAAGGAGATATACATATGATTTCTGTTTATATCATTTCTTTAAAAG (SEQ ID NO.1)
and a *PstI* site is added to the right primer:
5'CTGCAGTTAAACCTCTTTAGGGGTTTTTAAAGG (SEQ ID NO.2).

The amplified fragment is first cloned into pCR4Blunt-TOPO vector and then sub-cloned into the *XhoI* and *PstI* sites of pBBR3-SS plasmid to form pBBR3-SS-β-3GalT.

### Example 3. Production of COIV6S sialoside

The COIV6S sialoside presents the following formula:

Formula (V) Neu5Acα2-6Galβ1-4GlcNAcβ1-4GlcNAcβ1-4GlcNAcβ1-4GlcNAc

This formula corresponds to the general formula (I) with R1 = Galβ1-4, R2=H and n=3.

It is produced by fermentation of the strain SCH9, as described in example 1 for the strain SCH1.

The strain SCH9 is similar to SCH1 except that the plasmid pBS-nodC, encoding a chitinoligosaccharide synthase from *Azorhizobium caulinaudans,* is replaced by the plasmid pUC-nodC which encodes a chitinoligosaccharide synthase from Sinorhizobium meliloti and which was described by Samain *et al.* (1997).

### Example 4. Production of COII6S and COIII6S sialoside

The COII6S and COIII65 sialoside presents the following formula:

Formula (VI) Neu5Acα2-6Galβ1-4GlcNAcβ1-4GlcNAc

Formula (VII) Neu5Acα2-6Galβ1-4GlcNAcβ1-4GlcNAcβ1-4GlcNAc

Both formula of COII6S and COIII65 correspond to the general formula (I) with R1 = Galβ1-4, R2=H and n=1 or 2, respectively.

The COII6S and COIII65 sialosides are simultaneously produced by fermentation as described in example 1, except that the strain SCH1 is replaced by the strain SCH10. After the purification step on Dowex1, the two sialosides are separated by size exclusion chromatography on a HW40 column.

The strain SCH10 is similar to strain SCH1 except that plasmid pWKS-lgtB is replaced by the plasmid pWKS-lgtB-ChiA, which contained the additional *chiA* gene from Bacillus circulans WL12. The *chiA* gene has been previously shown to encodes a chitinase that cleaves chitinpentaose into chitinbiose with a transient accumulation of chitintriose (Cottaz and Samain, 2005).

The plasmid pWKS-lgtB-ChiA is constructed as follow: the 1.3 kb DNA segment containing the *chiA* gene is excised from pBBR1-ChiA (Cottaz and Samain 2007) by a *KpnI notI* digestion and cloned into the Xbal notI site of pWKS-lgtB in presence of Xbal/kpnl linker.

### Example 5. Production of LSTc sialoside (sialoside not comprised in the invention)

LSTc is a natural sialoside which is found in human milk and which is used as a reference compound. Its structure is:
Neu5Acα2-6Galβ1-4GlcNAcβ1-3Galβ1-4Glc

It can be produced by fermentation as described in example 1, except that the strain SCH1 is replaced by the strain LST1 and that lactose (5g/l) is added at the end of the exponential growth phase. The strain LST1 is similar to strain SCH14 expect that it does not contain the pBS-nodC plasmid.

### Example 6. Synthesis of multivalent sialosides

A simple approach based on the reductive amination of the amine groups of a natural polymer (ε-Poly-L-lysine) was used to link the terminal reducing end of different sialosides obtained in examples 1 to 5.

The grafting ratio, defined as the percentage of amine groups of ε-Poly-L-lysine which are substituted with a sialoside, can be controlled by the reaction conditions. As presented in table 2 below, it varies from 20 % to 100 %.

A short experimental protocol for obtaining ER15 is presented below:
In a 1.5 ml microtube, 0.4 mmol of sialoside (1 equivalent per amine) is solubilized in 500 µl of deionized water. Sonication is necessary to achieve perfect solubilization (solution 1). In another microtube 1.5 ml, 50 mg of polylysine are solubilized in 100 µl of deionized water (solution 2). The two solutions 1 and 2 are mixed and 100 µl of borate buffer (500 mM, pH 8.5) are added.

In a 1.5 ml microtube, under fume hood, 123.6 mg NaBH₃CN (2 mmol, 5 equivalents per oligosaccharide) is solubilized in 50 µl deionized water, and the resulting solution 3 is added dropwise to the previous mixture. The reaction mixture is vortexed and incubated at 40°C for 72 hours in a dry bath under fume hood.

The reaction mixture (1ml) is transferred to a 15 ml centrifuge tube. The microtube is washed with 1 ml deionized water which is transferred to the 15 ml tube (total volume 2 ml). After addition of 4 ml of 96% ethanol (2 volumes), the tube is centrifuged for 15 min at 9000 rpm. The supernatant (approx. 6 ml) is removed and the precipitate is taken up by 2 ml of a 1% NaCl solution. After the addition of 4 ml of 96% ethanol and a second centrifugation (15 min, 9000 rpm, 4°C), the precipitate is collected in 5 ml deionized water and purified on a preparative HW40 steric chromatography column (5 x 90 cm). Elution is performed with 100 mM ammonium carbonate at a flow rate of 2 ml/min.

After purification, the tubes containing the product are pooled. The product is evaporated to dryness to remove the ammonium carbonate, and then 3 ml of deionized and freeze-dried water is added.

To obtain the compounds ER59, 60 and 61, the number of oligosaccharide equivalent was modified: 0.75 equivalent per amine, 0.5 equivalent per amine and 0.25 equivalent per amine respectively.

The coupling rate is determined by nuclear magnetic resonance (NMR) 1H and Gel Permeation Chromatography (GPC) with Multi-Angle Light Scattering Detection (MALS).

**Table 2. Multivalent sialosides with a ε-Poly-L-lysine backbone**

| **Name** | **Sialoside name** | **Structure of sialoside** | **Grafting ratio** |
|---|---|---|---|
| ER13 | 6SL | NeuAcα2-6Galβ-4Glc | 61% |
| ER10 | LSTc | Neu5Aca2-6Galβ1-4GlcNAcβ1-3Galβ1-4Glc | 71% |
| ER100 | COIII6S | Neu5Acα2-6Galβ1-4GlcNAcβ1-4GlcNAcβ1-4GlcNAc | 80% |
| ER101 | COIV6S | Neu5Acα2-6Galβ1-4GlcNAcβ1-4GlcNAcβ1-4GlcNAcβ1-4GlcNAc | 73% |
| ER138 | 1-COV6S | Neu5Acα2-6Galβ1-3GlcNAcβ1-4GlcNAcβ1-4GlcNAcβ1-4GlcNAcβ1-4GlcNAc | 73% |
| ER15 | COV6S | Neu5Acα2-6Galβ1-4GlcNAcβ1-4GlcNAcβ1-4GlcNAcβ1-4GlcNAcβ1-4GlcNAc | 77% |
| ER59 | COV6S | Neu5Acα2-6Galβ1-4GlcNAcβ1-4GlcNAcβ1-4GlcNAcβ1-4GlcNAcβ1-4GlcNAc | 65% |
| ER60 | COV6S | Neu5Acα2-6Galβ1-4GlcNAcβ1-4GlcNAcβ1-4GlcNAcβ1-4GlcNAcβ1-4GlcNAc | 43% |
| ER61 | COV6S | Neu5Acα2-6Galβ1-4GlcNAcβ1-4GlcNAcβ1-4GlcNAcβ1-4GlcNAcβ1-4GlcNAc | 23% |

### Example 7. Hemagglutination inhibition assay (HI or HAI)

The HI technique consists in incubating decreasing doses of multivalent sialoside (possible inhibitors of the virus) with a fixed amount of virus. If there is recognition and binding, the viruses will attach themselves to the inhibitors.

Red blood cells are then added and the mixture is incubated. The free viruses will be able to recognize the sialic acids present on the surface of the red blood cells, and thus create a network (a red veil, representative of the hemagglutination process), while the viruses attached to the multivalent sialosides will not be able to bind to the red blood cells, which will sediment at the bottom of the well, creating a pellet (representative of hemagglutination inhibition).

The hemagglutination titer corresponds to the inverse of the highest dilution for which hemagglutination inhibition is observed. Dilutions are made at a ratio of 2, so a titer of <2 means that there is no hemagglutination inhibition observed from the first dilution at ½ while a titer of 4096, for example, means that there is hemagglutination inhibition until the 1/4096 dilution.

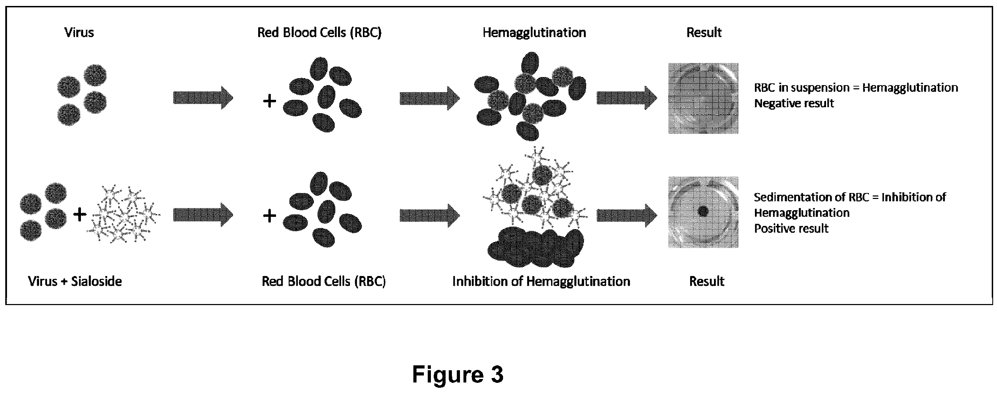

Figure 3 presents a recapitulative scheme of the HI technique.

For this HI assay, the viral strains used are: A/California/7/2009 H1N1, A/PortoRico/8/34 H1N1, A/Texas/50/12 H3N2, A/Moscow/10/99 H3N2, B/Massachusetts/2/2012 and B/Brisbane/60/08, at a dose of 4 HAU per well. The red blood cells used are 0.5% hen's red blood cells and 0.8% guinea pig red blood cells.

The table 3 below presents results obtained with multivalent sialosides listed in table 2. ER10 and ER13 are multivalent sialosides that are not included in formula (I) and therefore do not belong to the invention.

**Table 3. Results of HI assay with nine multivalent sialosides whose structures are presented in table 2**

| **Comp ounds** | **H1N1** | | | | **H3N2** | | | | **B** | | | |
|---|---|---|---|---|---|---|---|---|---|---|---|---|
| | A/California/ 07/09 | | A/PR/8/34 | | A/Texas/ 50/12 | | A/Moscou/10 /99 | | B/Massachu setts/2/201 2 | | B/Brisban e/60/08 | |
| | C* | GP* | C | GP | C | GP | C | GP | C | GP | C | GP |
| ER10 | 2 | < 2 | 16 | 2 | < 2 | < 2 | **128** | 8 | 16 | < 2 | < 2 | < 2 |
| ER13 | < 2 | < 2 | 2 | < 2 | < 2 | < 2 | < 2 | < 2 | < 2 | < 2 | < 2 | < 2 |
| ER15 | **128** | 8 | **4096** | **128** | < 2 | < 2 | 4 | < 2 | **64** | < 2 | < 2 | < 2 |
| ER59 | **128** | 16 | **2048** | **128** | < 2 | < 2 | 8 | 4 | **64** | 4 | < 2 | 8 |
| ER60 | **64** | 16 | **2048** | **256** | 2 | 8 | **128** | **64** | **256** | < 2 | < 2 | < 2 |
| ER61 | **128** | 16 | **1024** | **256** | 4 | < 2 | **512** | **128** | **256** | < 2 | < 2 | 4 |
| ER100 | < 2 | 4 | **64** | 16 | < 2 | < 2 | 8 | 4 | 16 | 8 | 4 | 8 |
| ER101 | 32 | 8 | **256** | **64** | < 2 | 2 | 8 | 4 | **64** | < 2 | < 2 | 2 |
| ER138 | 8 | < 8 | **64** | 16 | < 8 | < 8 | **64** | 16 | **256** | 32 | 8 | 32 |

| | | | | | | | | | | | | |
|---|---|---|---|---|---|---|---|---|---|---|---|---|
| * C: red blood cells from chicken; GP: red blood cells from guinea pig. | | | | | | | | | | | | |

Significant results are in bold characters.

The results presented in table 3 indicate that ER15 is the best performing compound in terms of HI on the H1N1 virus A/California/7/09 and A/PR/8/34 with titers of 128 and 4096 respectively, while ER10 or ER13 compounds present titers of 2 and 16 (ER10) and <2 and 2 (ER13).

These experiments highlight the importance of the presence of the GlcNAc motif, for their HI property and in particular the ER15 compound. Not all compounds have the same hemagglutination inhibition activity, or even no activity depending on the viral type (A or B) and/or the A virus subtype.

### Example 8. Microneutralization assay

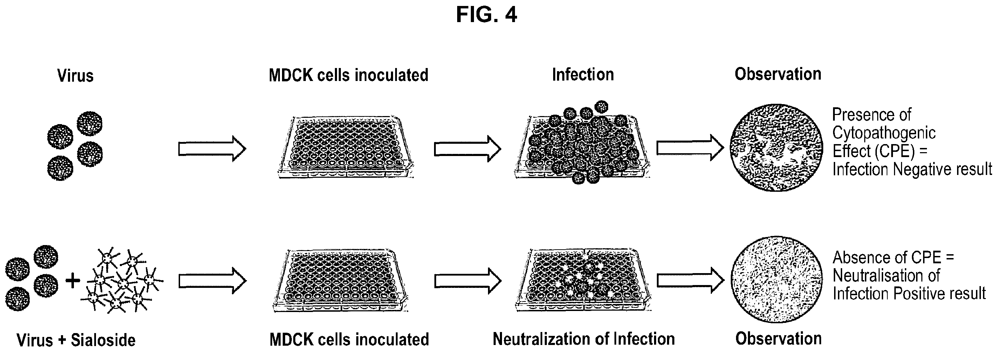

Microneutralization assay allows to quantify the inhibitory potential of an antiviral compound on an infectious virus. A schematic presentation of the technique is shown on figure 4.

This assay is carried out on a reference cell system for influenza viruses: Madin-Darby Canine Kidney Cells (MDCK) which present on their surface sialic acids either bound to galactose by an alpha 2.3 bond or by an alpha 2.6 bond. The virus strain used in this test was A/California/7/2009 H1N1 at a calibrated dose of 100 TCID50 in 50µL.

The technique consists of incubating decreasing doses of the tested compound with a fixed amount of virus for 1 hour. If recognized, the viruses will bind to the compounds. This virus/compound mixture is then added to the MDCK cells and incubated for 72 hours. The unbound viruses will be able to recognize the sialic acids present on the cell surface, and thus infect the cells (visualization of cytopathic effects), while the bound viruses to the sialylated compounds will not be able to attach themselves to the cells and thus will not infect them ("neutralization" of the infection).

The microneutralization titer is the inverse of the highest dilution for which a total absence of cytopathic effects is observed in at least 2 out of 4 wells. Dilutions are made at a ratio of 2 starting at 1:5, so a titer of <5 means that there is no neutralization of the infection from the first 1:5 dilution, whereas a titer of 160, for example, means that there is neutralization of infection up to the 1:160 dilution.

**Table 4. Results of microneutralization assay**

| **Reference** | **Sialoside** | **Molecular weight** | **Grafting rate %** | **0,1 mM solution mg/ml*** | **Results** |
|---|---|---|---|---|---|
| | | | | | **Titre of MN** |
| **Polylysine** | none (negative control) | 4754 | | 0,48 | < 5 |
| **ER15** | COV6S | 40059 | 65 | 4,01 | 160 |
| **ER59** | COV6S | 42612 | 69 | 4,25 | 80 |
| **ER60** | COV6S | 28875 | 44 | 2,89 | 80 |
| **ER61** | COV6S | 17886 | 24 | 1,79 | 40 |
| ***concentration of the sample in mg/ml used to obtain stock solutions at 0,1 mM** | | | | | |

ER15 multivalent sialoside presents the best neutralizing activity on the virus strain A/California/7/2009 H1N1.

### Example 9. Assay of multivalent sialosides of the invention on human cell line A549

The objective of this assay is the evaluation of the performance of ER15 as an antiviral agent, on a human respiratory cell line A549 (from human lung carcinoma) under different treatment conditions (therapeutic/post-infection, prophylactic/pre-infection treatment), at different concentrations and with multiple viral doses (dose-effect).

A549 cells are cultured in DMEM + SVF 10% + L-glutamine 2mM + Penicillin (100U/ml) + Streptomycin (100µg/ml) in an incubator at 37° C and 5% CO2. For infection and evaluation of compounds, cells are inoculated in 12-well plates in DMEM + L-glutamine 2mM + Penicillin (100U/ml) + Streptomycin (100µg/ml)+ Bovine Trypsin (T6763 Sigma)0.5µg/ml.

The A/California/7/2009 H1N1 viral strain is used at two different infection multiplicity (MOI): 0.1 and 0.01. This strain possesses a D225G mutated hemagglutinin, giving it greater affinity for its cellular receptor.

Another viral strain A/Lyon/969/09 H1N1 is used at three different MOIs: 1; 0.1 and 0.01. This strain does not present the D225G mutation and has a lower affinity for the alpha 2-6 sialic acids of the cellular receptors.

The lyophilized compounds are mixed with water at 0.1mM, filtered on 0.22µM and stored at -20°C. The compound used as a negative control is polylysine alone (PL).

After treatment and infection of A549 cells, supernatant samples are taken at D1, D2 and D3 post-infection to quantify viral production by (i) titration of the number of infectious particles per ml (TCID50/ml) on MDCK cells and (ii) quantification of the viral genome (RT-qPCR).

### A - Therapeutic properties of ER15 vs. polylysine alone (PL) against H1N1 strain

Potential therapeutic effects of the ER15 compound are assayed with three post-infection treatments (at D0 (1 hour pi), D1 and D2). The A/California/7/09 H1N1 virus is incubated for 1 hour at 37°C on the cells and then removed.

Medium with ER15 is added - ER15 is present at 5 different concentrations (20; 10; 5; 2.5 and 1.25µM) vs untreated conditions (NT: untreated and PL: delivery of increasing amounts of polylysine). Several samples of the cell supernatant are taken at 3 different times for viral quantification.

The results obtained, shown in figure 5, indicate that ER15 treatment administered at 1, 24 and 48 hour(s) post-infection provides a 5- to 6-log reduction in the A/California/7/09 H1N1 titer. A dose-dependent effect depending on the treatment concentration is observed when the viral inoculum is at an MOI of 0.1. At an MOI of 0.01, all ER15 concentrations tested (20; 10; 5; 2.5 and 1.25µM) allow maximum viral load reduction as early as 24hpi.

The results obtained in RT-qPCR are concordant and indicate a significant inhibition of infection.

### B- Therapeutic properties of ER15 vs. polylysine alone (PL) against H3N2 strain

Experimental conditions are the same than for (A), except that the A/Texas/50/2012 H3N2 virus is incubated for 1 hour at 37°C on the cells and then removed.

The results obtained, shown in figure 6, indicate:
- a significant reduction in the infectious titer (approximately 3-log) is observed with a ER15 treatment, administered at 1, 24 and 48 hour(s) post-infection, at a dose of 20µM, and for the 2 MOIs tested, i.e. 0.1 and 0.01.
- ER15 treatment at concentrations of 1.25 to 10µM does not significantly reduce the infectious viral load for this H3N2 strain.

These results are consistent with those obtained in HIA where the titer was <2.

Therefore, a significant effect against H3N2 strain is observed, in a dose-dependent manner.

The results obtained in RT-qPCR are concordant and also show a significant viral load reduction for the treatment at 20µM only.

Overall, the HIA and efficacy results in A549 cells, show a lower affinity/performance of ER15 against the A/Texas/50/2012 H3N2 strain compared to the A/California/7/09 H1N1.

### C- Therapeutic properties of ER15 vs. polylysine alone (PL) against A/California/7/09 H1N1 strain, with only one administration post infection

Experimental conditions are the same than for (A), except that ER15 is administered to infected cells, via addition into the medium, only once at D0, 1 hour pi. 3 different concentrations are tested: 20; 5 and 1,25µM.

The results obtained, shown in figure 7, indicate that ER15 treatment administered at 1 hour post-infection is sufficient to drastically reduce the infectious viral load whatever the starting MOI (0.1 or 0.01). A reduction of at least 2 log is observed at 24 hpi, and up to more than 5 log at 72 hpi. The effect is therefore persistent with a single administration (1hpi) and a dose-effect is observed with the MOI 0.1.

### D- Therapeutic properties of ER15 vs. polylysine alone (PL) against A/California/7/09 H1N1 strain, with only one administration concomitant with the viral infection

Experimental conditions are the same than for (A), except that ER15 is administered to cells, via addition into the medium, only once at D0, with the virus. 3 different concentrations are tested: 20; 5 and 1,25µM.

The results obtained, shown in figure 8, indicate that ER15 treatment administered at the same time as the virus (during the infection step) allows a significant decrease in the infectious viral load (up to 3-log), in particular in the early stages (24 and 48h pi). In the condition of MOI 0.01, the effect is maintained over time (between 4 and 5 log). A dose-dependent effect of ER15 concentration is observed as well as a dose-dependent effect of MOI.

### E- Prophylactic properties of ER15 vs. polylysine alone (PL) against A/California/7/09 H1N1 strain,

The objective here is to evaluate the prophylactic effect of the ER15 compound with a single treatment carried out one day before infection, at 3 different concentrations (20; 5 and 1.25µM).

At the time of infection, the A/California/7/09 H1N1 virus is added directly into the medium already containing the compound. After 5 hours of incubation, the virus and compound containing medium is removed and replaced by fresh medium.

Samples are taken at D1, D2 and D3 for infectious viral quantification.

The results obtained, shown in figure 9, indicate that a single prophylactic treatment with ER15, carried out 24 hours before the infection, significantly reduces the infectious viral load over time : between 2 and 3 log from 24 h pi and up to 5 log reduction, depending on the MOI. The effect is maintained over time (between 4 and 5 log). A dose-dependent effect of ER15 concentration and MOI is clearly observed.

### F- Therapeutic properties of ER15 vs. polylysine alone (PL) against H1N1 A/Lyon/969/09 strain

Experimental conditions are the same than for (A), except that the H1N1 A/Lyon/969/09 strain virus is incubated for 1 hour at 37°C on the cells and then removed.

The results obtained, shown in figure 10, indicate that ER15 significantly reduces the infectious viral load of the A/Lyon/969/09 H1N1 strain. As expected, results on the Lyon strain are less good than those with the California strain, which presents the D225G mutation.

### Example 10. Evaluation of the ER15 compound in an in vitro physiological model based on reconstructed human respiratory epithelia of nasal origin

Epithelia are grown on inserts placed in 24 wells plates at the air/liquid interface. Thus, the cells constituting the basal pole are in contact with the culture medium and the cells constituting the apical pole are in contact with air. The culture medium is Mucilair (Epithelix) and the epithelia are derived from nasal swab from a donor pool (Epithelix). They are maintained in culture in an incubator at 37°C with 5% CO2.

The infection by the A/California/7/2009 H1N1 strain occurs at the apical pole in 150µl of OptiMEM medium, at an infection multiplicity of 0.1, for 1 hour at 37°C and 5% CO2. Then, the viral suspension is removed and a wash is performed. After carefully removing the entire medium at the apical pole, the treatment is performed at 1 hpi in 10µl at 3 different concentrations (50; 20 and 5µM).

The lyophilized compound ER15 is mixed with:
- water,
- phosphate buffer (2g/L glycerin 9g/L NaCl 10mM pH = 7.9) and
- citrate buffer (2g/L glycerin 9g/L NaCl 10mM pH = 6.0)
at a concentration of 0.1mM.

A negative control is polylysine (PL) diluted in water at 0.1mM.

Some cells are untreated (NT) and/or mock (uninfected).

Trans-epithelial electrical resistance (TEER) measurements are performed daily to monitor the integrity of the epithelium. Samples are collected at the apical pole at D1, D2 and D3 to quantify viral production, by titration of the number of infectious particles per ml (TCID50/ml - Fig 11C) and quantification of the viral genome by RT-qPCR (Fig 11D).

In parallel, the cytotoxicity of ER15 is evaluated under mock-infected conditions under the same treatment conditions. The viral inoculum volume was replaced here by OptiMEM medium. Samples are taken daily in basal medium to quantify the release of lactate dehydrogenase (LDH, released in case of cytotoxicity). The results obtained, shown in figure 11B, indicate that the ER15 compound is not cytotoxic up to at least 50µM on epithelium.

Furthermore, ER15 at 50µM significantly reduces the infectious viral load in epithelium (figures 11C and 11D). The phosphate and citrate buffers seem to allow a better efficacy of ER15 at 20µM, compared to the solvent water.

These results are confirmed in RT-qPCR as shown in figure 11D. TEER measurements are also consistent with the overall results.

### Example 11. Therapeutic properties of ER61 vs. polylysine alone (PL) against A/California/7/09 H1N1 strain

Potential therapeutic effects of the ER61 compound are assayed with three post-infection treatments (at D0 (1 hour pi), D1 and D2). The A/California/7/09 H1N1 virus is incubated for 1 hour at 37°C on the A549 cells, at multiplicity of infection of 0.1 or 0.01, and then removed.

Experimental conditions are the same than for example 9 (A), except that the ER61 compound is used.

ER61 is tested at 5 different concentrations (20; 10; 5; 2.5 and 1.25 µM) vs untreated conditions (NT: untreated and PL: delivery of increasing amounts of polylysine). Several samples of the cell supernatant are taken at 3 different times for viral quantification.

The results obtained, shown in figure 12, indicate that ER61 treatment administered at 1, 24 and 48 hour(s) post-infection provides up to 5-log reduction in the A/California/7/09 H1N1 titer. A dose-dependent effect depending on the treatment concentration is observed when the viral inoculum is at an MOI of 0.1. At an MOI of 0.01, all ER61 concentrations tested (20; 10; 5; 2.5 and 1.25µM) allow maximum viral load reduction as early as 24h pi.

### Example 12. Therapeutic properties of ER15 and ER61 compounds against recent strains

The tested "recent" strains which are entered in vaccine composition are the following: A/Michigan/45/2015 H1N1; A/Kansas/14/2017 H3N2; and B/Phuket/3073/2013.

Potential therapeutic effects of the ER15 and ER61 compounds are assayed with three post-infection treatments (at D0 (1 hour pi), D1 and D2). The virus is incubated for 1 hour at 37°C on the A549 cells, at a multiplicity of infection of 0.1, and then removed.

Multivalent sialosides ER15 and ER61 are tested at 3 different concentrations (20; 5; and 1.25 µM) vs untreated conditions (NT: untreated). Several samples of the cell supernatant are taken at 3 different times (24, 48 and 72 hours pi) for viral quantification.

The results obtained, shown in figures 13A, 13B and 13C, indicate that ER15 and ER61 treatments administered at 1, 24 and 48 hour(s) post-infection are equivalent. A dose-dependent effect depending on the treatment concentration is observed. At a concentration of 20 µM, both compounds ER15 and ER61 present a significant antiviral effect on the three viral strains.

### Example 13. Therapeutic effects of the ER15 compound against recombinant oseltamivir- and baloxavir resistant H1N1 viruses

Three recombinant H1N1 viruses were generated by genetic reverse with the A/Lyon/969/09 H1N1 genetic backbone harboring either the H275Y mutation in neuraminidase (conferring resistance to oseltamivir), or the I38T mutation in the polymerase PA subunit (conferring resistance to baloxavir), or both (H275Y + 138T). Potential therapeutic effects of the ER15 compound are assayed with three post-infection treatments (at D0 (1 hour pi), D1 and D2). The recombinant resistant H1N1 viruses are incubated for 1 hour at 37°C on the A549 cells, at multiplicity of infection of 0.1, and then removed by washing. Experimental conditions are the same than for example 9 (A), except that the viruses are used. ER15 is tested at 5 different concentrations (20; 10; 5; 2.5 and 1.25 µM) vs untreated conditions (NT: untreated and PL: delivery of increasing amounts of polylysine). Several samples of the cell supernatant are taken at 3 different times for viral quantification.

The results obtained, shown in figure 14A, indicate that ER15 treatment administered at 1, 24 and 48 hour(s) post-infection provides up to 2-log reduction in the H1N1 I38T titer. A dose-dependent effect depending on the treatment concentration is observed Similar results are obtained with the H1N1 H275Y and the double resistant H1N1 (I28T + H275Y viruses, as illustrated in figure 14B and figure 14C, respectively.

### REFERENCES

### PATENTS

WO 2007/101862

### NON-PATENT LITERATURE

Matrosovich M, Klenk HD. Natural and synthetic sialic acid-containing inhibitors of influenza virus receptor binding. Rev Med Virol. 2003 Mar-Apr;13(2):85-97.
Nycholat CM, McBride R, Ekiert DC, Xu R, Rangarajan J, Peng W, Razi N, Gilbert M, Wakarchuk W, Wilson IA, Paulson JC. Recognition of sialylated poly-N-acetyllactosamine chains on N- and O-linked glycans by human and avian influenza A virus hemagglutinins. Angew Chem Int Ed Engl. 2012 May 14;51(20):4860-3.
Yang H, Carney PJ, Chang JC, Guo Z, Villanueva JM, Stevens J. Structure and receptor binding preferences of recombinant human A(H3N2) virus hemagglutinins. Virology. 2015 Mar;477:18-31.
Bych K, Mikš MH, Johanson T, Hederos MJ, Vigsnæs LK, Becker P. Production of HMOs using microbial hosts - from cell engineering to large scale production. Curr Opin Biotechnol. 2019 Apr;56:130-137.
Jill E. Kingery-Wood, Kevin W. Williams, George B. Sigal, and George M. Whitesides. The agglutination of erythrocytes by influenza virus is strongly inhibited by liposomes incorporating an analog of sialyl gangliosides. Journal of the American Chemical Society 1992 114 (18), 7303-7305
Reuter JD, Myc A, Hayes MM, Gan Z, Roy R, Qin D, Yin R, Piehler LT, Esfand R, Tomalia DA, Baker JR Jr. Inhibition of viral adhesion and infection by sialic-acid-conjugated dendritic polymers. Bioconjug Chem. 1999 Mar-Apr;10(2):271-8.
Gambaryan AS, Boravleva EY, Matrosovich TY, Matrosovich MN, Klenk HD, Moiseeva EV, Tuzikov AB, Chinarev AA, Pazynina GV, Bovin NV. Polymer-bound 6' sialyl-N-acetyllactosamine protects mice infected by influenza virus. Antiviral Res. 2005 Dec;68(3):116-23.
Myco Umemura, Yutaka Makimura, Masae Itoh, Takeshi Yamamoto, Toshiki Mine, Seiji Mitani, Ichiro Simizu, Hisashi Ashida, Kenji Yamamoto. One-step synthesis of efficient binding-inhibitor for influenza virus through multiple addition of sialyloligosaccharides on chitosan. Carbohydrate Polymers, Volume 81, Issue 2, 2010, Pages 330-334.
Li X, Wu P, Gao GF, Cheng S. Carbohydrate-functionalized chitosan fiber for influenza virus capture. Biomacromolecules. 2011 Nov 14;12(11):3962-9. doi: 10.1021/bm200970x. Epub 2011 Oct 13. PMID: 21978096.
Papp I, Sieben C, Ludwig K, Roskamp M, Böttcher C, Schlecht S, Herrmann A, Haag R. Inhibition of influenza virus infection by multivalent sialic-acid-functionalized gold nanoparticles. Small. 2010 Dec 20;6(24):2900-6.
Matrosovich M, Herrler G, Klenk HD. Sialic Acid Receptors of Viruses. Top Curr Chem. 2015;367:1-28
Samain E, Drouillard S, Heyraud A, Driguez H, Geremia RA. Gram-scale synthesis of recombinant chitooligosaccharides in Escherichia coli. Carbohydr Res. 1997 Jul 11;302(1-2):35-42.
Bettler E, Samain E, Chazalet V, Bosso C, Heyraud A, Joziasse DH, Wakarchuk WW, Imberty A, Geremia AR. The living factory: in vivo production of N-acetyllactosamine containing carbohydrates in E. coli. Glycoconj J. 1999 Mar;16(3):205-12.
Cottaz S, Samain E. Genetic engineering of Escherichia coli for the production of NI,NII-diacetylchitobiose (chitinbiose) and its utilization as a primer for the synthesis of complex carbohydrates. Metab Eng. 2005 Jul;7(4):311-7.
Fierfort N, Samain E. Genetic engineering of Escherichia coli for the economical production of sialylated oligosaccharides. J Biotechnol. 2008 Apr 30;134(3-4):261-5.
Richard E, Pifferi C, Fiore M, Samain E, Le Gouëllec A, Fort S, Renaudet O, Priem B. Chemobacterial Synthesis of a Sialyl-Tn Cyclopeptide Vaccine Candidate. Chembiochem. 2017 Sep 5;18(17):1730-1734.
Priem B, Gilbert M, Wakarchuk WW, Heyraud A, Samain E. A new fermentation process allows large-scale production of human milk oligosaccharides by metabolically engineered bacteria. Glycobiology. 2002 Apr;12(4):235-40.

## Claims

1. Synthetic sialoside presenting the formula (I):
Neu5Ac-α2-6-R1(R2)[GlcNAcβ1-4]ₙ-GlcNAc Formula (I)
wherein:
• GlcNAc is N-acetylglucosamine;
• GlcNAcβ1-4 is a N-acetylglucosamine unit linked with a β1-4 link;
• n is superior or equal to 1;
• R1 is a glycan structure comprising at least one galactose (Gal), and where the glycan structure has a main chain comprising five monosaccharides at most; and
• R2 is chosen among the following groups: H, fucose linked with a α1-3 link (Fucα1-3) or a α1-4 link (Fucα1-4).

2. Synthetic sialoside according to claim 1, wherein R1 is a glycan structure with a main chain comprising one, two or three monosaccharides.

3. Synthetic sialoside according to claim 1 or 2, wherein R1 is a glycan structure where at least one galactose is linked at one of the extremities of the main chain.

4. Synthetic sialoside according to anyone of claims 1 to 3, wherein R1 is chosen among the following groups: Galβ1-4, Galβ1-3, Galβ1-4GlcNAcβ1-3Galβ1-4 and Galβ1-4 (Fucα1-3)GlcNAcβ1-3Galβ1-4.

5. Synthetic sialoside according to anyone of claims 1 to 4, wherein R2 is H.

6. Synthetic sialoside according to anyone of claims 1 to 5 presenting the formula (II):
Neu5Acα2-6Galβ1-4GlcNAcβ1-4GlcNAcβ1-4GlcNAcβ1-4GlcNAcβ1-4GlcNAc

7. Multivalent sialoside comprising a support with multiple synthetic sialosides according to anyone of claims 1 to 6.

8. Multivalent sialoside according to claim 7, wherein the support consists of liposomes, dendrimers, polymers or nanoparticules.

9. Multivalent sialoside according to claim 7 or 8, wherein the support consists of Polylysine (ε-Poly-L-lysine).

10. Multivalent sialoside according to anyone of claims 7 to 9, wherein the grafting rate of sialosides on the support is comprised between 20% and 100%.

11. Pharmaceutical composition comprising, in a pharmaceutically acceptable vehicle, at least one multivalent sialoside according to anyone of claims 7 to 10.

12. Pharmaceutical composition according to claim 11, wherein it is in a form suitable for administration by inhalation.

13. Medical device comprising at least one multivalent sialoside according to anyone of claims 9 to 10.

14. Multivalent sialoside according to anyone of claims 7 to 10, or pharmaceutical composition according to claim 11 or 12, for its use as a medicament.

15. Multivalent sialoside according to anyone of claims 7 to 10, or pharmaceutical composition according to claim 11 or 12, for its use in the prevention and/or the treatment of an infection due to a virus having an affinity for sialic acid.

16. Multivalent sialoside or pharmaceutical composition for its use according to claim 15, wherein the virus is an influenza virus, in particular a human, equin, porcine or avian influenza virus.

17. Multivalent sialoside or pharmaceutical composition for its use according to claim 16, wherein the influenza virus is human, and is chosen among strains H1N1, H3N2 and B.

## Patentansprüche

1. Synthetisches Sialosid, das der Formel (I) entspricht:
Neu5Ac-α2-6-R1(R2)[GlcNAcβ1-4]ₙ-GlcNAc Formel (I)
worin gilt:
• GlcNAc steht für N-Acetylglucosamin;
• GlcNAcβ1-4 ist eine N-Acetylglucosamin-Einheit, die über eine β1-4-Bindung verknüpft ist;
• n ist größer oder gleich 1;
• R1 ist eine Glykanstruktur, die mindestens eine Galaktose (Gal) umfasst und deren Hauptkette höchstens fünf Monosaccharide enthält; und
• R2 ist gewählt aus den folgenden Gruppen: H, Fucose mit einer α1-3-Bindung (Fucα1-3) oder einer α1-4-Bindung (Fucα1-4).

2. Synthetisches Sialosid nach Anspruch 1, wobei R1 eine Glykanstruktur mit einer Hauptkette aus einem, zwei oder drei Monosacchariden ist.

3. Synthetisches Sialosid nach Anspruch 1 oder 2, wobei R1 eine Glykanstruktur ist, bei der mindestens eine Galaktose an einem Ende der Hauptkette gebunden ist.

4. Synthetisches Sialosid nach einem der Ansprüche 1 bis 3, wobei R1 aus den folgenden Gruppen gewählt ist: Galβ1-4, Galβ1-3, Galβ1-4GlcNAcβ1-3Galβ1-4 und Galβ1-4(Fucα1-3)GlcNAcβ1-3Galβ1-4.

5. Synthetisches Sialosid nach einem der Ansprüche 1 bis 4, wobei R2 H ist.

6. Synthetisches Sialosid nach einem der Ansprüche 1 bis 5, das der Formel (II) entspricht: Neu5Acα2-6Galβ1-4GlcNAcβ1-4GlcNAcβ1-4GlcNAcβ1-4GlcNAcβ1-4GlcNAc.

7. Multivalentes Sialosid, das einen Träger mit mehreren synthetischen Sialosiden gemäß einem der Ansprüche 1 bis 6 umfasst.

8. Multivalentes Sialosid nach Anspruch 7, wobei der Träger aus Liposomen, Dendrimeren, Polymeren oder Nanopartikeln besteht.

9. Multivalentes Sialosid nach Anspruch 7 oder 8, wobei der Träger aus Polylysin (ε-Poly-L-lysin) besteht.

10. Multivalentes Sialosid nach einem der Ansprüche 7 bis 9, wobei die Verknüpfungsrate der Sialoside auf dem Träger zwischen 20 % und 100 % liegt.

11. Pharmazeutische Zusammensetzung, die mindestens ein multivalentes Sialosid gemäß einem der Ansprüche 7 bis 10 in einem pharmazeutisch verträglichen Träger umfasst.

12. Pharmazeutische Zusammensetzung nach Anspruch 11, wobei sie in einer Form zur Verabreichung durch Inhalation vorliegt.

13. Medizinisches Gerät, das mindestens ein multivalentes Sialosid gemäß einem der Ansprüche 9 bis 10 umfasst.

14. Multivalentes Sialosid gemäß einem der Ansprüche 7 bis 10 oder pharmazeutische Zusammensetzung gemäß Anspruch 11 oder 12 zur Verwendung als Arzneimittel.

15. Multivalentes Sialosid gemäß einem der Ansprüche 7 bis 10 oder pharmazeutische Zusammensetzung gemäß Anspruch 11 oder 12 zur Verwendung bei der Vorbeugung und/oder Behandlung einer Infektion durch ein Virus mit Affinität zu Sialinsäure.

16. Multivalentes Sialosid oder pharmazeutische Zusammensetzung zur Verwendung gemäß Anspruch 15, wobei das Virus ein Influenzavirus ist, insbesondere ein humanes, equines, porcines oder aviäres Influenzavirus.

17. Multivalentes Sialosid oder pharmazeutische Zusammensetzung zur Verwendung gemäß Anspruch 16, wobei das Influenzavirus ein humanes Virus ist, ausgewählt aus den Stämmen H1N1, H3N2 und B.

## Revendications

1. Sialoside synthétique présentant la formule (I) :
Neu5Ac-α 2-6-R1(R2)[GlcNAcβ 1-4]ₙ-GlcNAc Formule (I)
dans laquelle :
• GlcNAc est la N-acétylglucosamine ;
• GlcNAcβ1-4 est une unité de N-acétylglucosamine liée par un lieur β1-4 ;
• n est supérieur ou égal à 1 ;
• R1 est une structure glycane comprenant au moins un galactose (Gal), et dans laquelle la structure glycane a une chaîne principale comprenant au plus cinq monosaccharides ; et
• R2 est choisi parmi les groupes suivants : H, fucose lié par une liaison α1-3 (Fucα1-3) ou une liaison α1-4 (Fucα1-4).

2. Sialoside synthétique selon la revendication 1, dans lequel R1 est une structure glycane avec une chaîne principale comprenant un, deux ou trois monosaccharides.

3. Sialoside synthétique selon la revendication 1 ou 2, dans lequel R1 est une structure glycane où au moins un galactose est lié à l'une des extrémités de la chaîne principale.

4. Sialoside synthétique selon l'une quelconque des revendications 1 à 3, dans lequel R1 est choisi parmi les groupes suivants : Galβ1-4, Galβ1-3, Galβ1-4GlcNAcβ1-3Galβ1-4 et Galβ1-4 (Fucα1-3)GlcNAcβ1-3Galβ1-4.

5. Sialoside synthétique selon l'une quelconque des revendications 1 à 4, dans lequel R2 est H.

6. Sialoside synthétique selon l'une quelconque des revendications 1 à 5, présentant la formule (II) :
Neu5Acα2-6Galβ1-4GlcNAcβ1-4GlcNAcβ1-4GlcNAcβ1-4GlcNAcβ1-4GlcNAc

7. Sialoside multivalent comprenant un support avec de multiples sialosides synthétiques selon l'une quelconque des revendications 1 à 6.

8. Sialoside multivalent selon la revendication 7, dans lequel le support consiste en des liposomes, des dendrimères, des polymères ou des nanoparticules.

9. Sialoside multivalent selon la revendication 7 ou 8, dans lequel le support consiste en la Polylysine (ε-Poly-L-lysine).

10. Sialoside multivalent selon l'une quelconque des revendications 7 à 9, dans lequel le taux de greffage des sialosides sur le support est compris entre 20 % et 100 %.

11. Composition pharmaceutique comprenant, : dans un véhicule pharmaceutiquement acceptable, au moins un sialoside multivalent selon l'une quelconque des revendications 7 à 10.

12. Composition pharmaceutique selon la revendication 11, dans laquelle elle se présente sous une forme adaptée à l'administration par inhalation.

13. Dispositif médical comprenant au moins un sialoside multivalent selon l'une quelconque des revendications 9 à 10.

14. Sialoside multivalent selon l'une quelconque des revendications 7 à 10, ou une composition pharmaceutique selon la revendication 11 ou 12, pour son utilisation comme médicament.

15. Sialoside multivalent selon l'une quelconque des revendications 7 à 10, ou une composition pharmaceutique selon la revendication 11 ou 12, pour son utilisation dans la prévention et/ou le traitement d'une infection due à un virus ayant une affinité pour l'acide sialique.

16. Sialoside multivalent ou composition pharmaceutique pour son utilisation selon la revendication 15, dans laquelle le virus est un virus grippal, en particulier un virus grippal humain, équin, porcin ou aviaire.

17. Sialoside multivalent ou composition pharmaceutique pour son utilisation selon la revendication 16, dans laquelle le virus de la grippe est humain et est choisi parmi les souches H1N1, H3N2 et B.
